Europäisches Patentamt

European Patent Office

Office européen des brevets

(11) Veröffentlichungsnummer: **0 382 174 B1**

(12) **EUROPÄISCHE PATENTSCHRIFT**

(45) Veröffentlichungstag der Patentschrift: **09.08.95**

(51) Int. Cl.⁶: **C12N 9/64**, C12N 15/58, A61K 38/49

(21) Anmeldenummer: **90102329.1**

(22) Anmeldetag: **06.02.90**

(54) **Gewebs-Plasminogenaktivator-Derivat.**

(30) Priorität: **07.02.89 DE 3903581**

(43) Veröffentlichungstag der Anmeldung:
**16.08.90 Patentblatt 90/33**

(45) Bekanntmachung des Hinweises auf die
Patenterteilung:
**09.08.95 Patentblatt 95/32**

(84) Benannte Vertragsstaaten:
**AT BE CH DE DK ES FR GB GR IT LI LU NL SE**

(56) Entgegenhaltungen:
**EP-A- 0 219 874**
**EP-A- 0 245 100**
**EP-A- 0 297 066**
**EP-A- 0 302 456**

(73) Patentinhaber: **BOEHRINGER MANNHEIM GMBH**

**D-68298 Mannheim (DE)**

(72) Erfinder: **Stern, Anne, Dr. rer nat**
**Karwendelstrasse 10**
**D-8122 Penzberg (DE)**
Erfinder: **Kohnert, Ulrich, Dr. rer. nat.**
**Heubachweg 6**
**D-8121 Habach (DE)**
Erfinder: **Rudolph, Rainer, Dr. rer. nat.**
**Färbergasse 19**
**D-8120 Weilheim (DE)**
Erfinder: **Fischer, Stephan, Dr. rer. nat.**
**Jakobifeldweg 11**
**D-8121 Polling (DE)**
Erfinder: **Martin, Ulrich, Dr. med.**
**D 3,4**
**D-6800 Mannheim 1 (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

**Beschreibung**

Die Erfindung betrifft ein neues t-PA-Derivat, eine DNA-Sequenz, welche für das neue t-PA-Derivat kodiert, Expressionsplasmide, welche eine für das t-PA-Derivat kodierende DNA-Sequenz aufweisen sowie ein Verfahren zur Herstellung derartiger Plasmide, Verfahren zur Herstellung des t-PA-Derivats und ein Mittel zur Gerinnselauflösung, enthaltend das t-PA-Derivat.

Geronnenes Blut enthält als Hauptkomponente der Proteinmatrix polymeres Fibrin. Fibrin wird durch ein fibrinolytisches System unter physiologischen Bedingungen in einer Reaktionskaskade aufgelöst, die der der Blutgerinnung ähnelt. Die zentrale Reaktion ist hierbei die Aktivierung von Plasminogen zu Plasmin, die z.B. durch den Gewebsplasminogenaktivator t-PA (tissue type plasminogen activator) bewirkt wird. Plasmin wiederum löst Fibrin, das die Hauptkomponente der Proteinmatrix von geronnenem Blut ist. Die enzymatische Aktivität von natürlichem oder aus Eukaryonten gentechnologisch gewonnenem t-PA, nämlich die katalytische Aktivierung von Plasminogen zu Plasmin, ist in Abwesenheit von Fibrin oder Fibrinogen-Spaltprodukten sehr gering, kann jedoch bei Anwesenheit dieser Proteine deutlich gesteigert werden, nämlich um mehr als den Faktor 10.

t-PA wird im Blut durch vorhandene Proteasen in eine A-und eine B-Kette gespalten. Die beiden Teilketten bleiben über eine Cysteinbrücke verbunden. Die Stimulierbarkeit der Aktivität des t-PA ist ein entscheidender Vorteil gegenüber anderen bekannten Plasminogen-Aktivatoren, wie z.B. Urokinase oder Streptokinase (vgl. z.B. M. Hoylaerts et al., J. Biol. Chem. 257 (1982), 2912-2919; W. Nieuwenhuizen et al., Biochem. Biophys. Acta, 755 (1983), 531-533).

Der Wirkungsmechanismus von t-PA in vivo ist beispielsweise in Korniger und Collen, Thromb. Hämostasis 46 (1981), 561-565, beschrieben. Die auf die Fibrinoberfläche focussierte Aktivität des Enzyms läßt es als geeignetes Mittel zur Bekämpfung von pathologischen Aderverschlüssen (z.B. beim Herzinfarkt) erscheinen, was durch klinische Versuche größtenteils bestätigt werden konnte (Collen et. al., Circulation 70 (1984), 1012; Circulation 73 (1986), 511).

Als Nachteil von t-PA muß jedoch seine rasche Abnahme der Plasmakonzentration (Clearance) angesehen werden. Die Folge daraus ist, daß eine relativ große Menge an t-PA erforderlich ist, um in vivo eine effektive Lysierung von Thromben zu erzielen. Die hohen Behandlungsdosen wiederum haben Nebenwirkungen, wie z.B. Blutungen, zur Folge.

In der EP 0 196 920 ist ein natürliches Abbauprodukt von t-PA beschrieben, welches nur noch die Kringel II- und Protease-Domänen enthält und dessen N-Terminus mit Alanin 160 beginnt (Zählweise nach der von Pennica et al. in Nature 301 (1983) 214-221 angegebenen Aminosäuresequenz).

Die Clearance-Rate dieses t-PA-Abbauprodukts ist jedoch nicht wesentlich von der des natürlichen t-PA verschieden. Erst durch eine chemische Modifikation des katalytischen Bereichs durch Anbringen einer blockierenden Gruppe kann hier eine Verbesserung erreicht werden.

Aufgabe der Erfindung ist es daher, t-PA derart zu verändern, daß das entstandene Derivat eine stark reduzierte Clearance-Rate und damit eine verlängerte Halbwertszeit im Blutplasma aufweist. Es sollen hierbei die Thromben-lysierende Wirkung sowie die Stimulierbarkeit durch Fibrin erhalten bleiben.

Gegenstand der Erfindung ist daher ein Gewebsplasminogen-Aktivator (t-PA-Derivat), der dadurch gekennzeichnet ist, daß er nicht glykosyliert ist und aus der folgenden Aminosäuresequenz besteht:

```
        (M)

  1   SYQGNSDCYF  GNGSAYRGTH  SLTESGASCL  PWNSMILIGK  VYTAQNPSAQ

 51   ALGLGKHNYC  RNPDGDAKPW  CHVLKNRRLT  WEYCDVPSCS  TCGLRQYSQP

101   QFRIKGGLFA  DIASHPWQAA  IFAKHRRSPG  ERFLCGGILI  SSCWILSAAH

151   CFQERFPPHH  LTVILGRTYR  VVPGEEEQKF  EVEKYIVHKE  FDDDTYDNDI

201   ALLQLKSDSS  RCAQESSVVR  TVCLPPADLQ  LPDWTECELS  GYGKHEALSP

251   FYSERLKEAH  VRLYPSSRCT  SQHLLNRTVT  DNMLCAGDTR  SGGPQANLHD

301   ACQGDSGGPL  VCLNDGRMTL  VGIISWGLGC  GQKDVPGVYT  KVTNYLDWIR

351   DNMRP
```

die am Aminoende, d.h. an der Aminosäure Nr. 1 = S noch durch M verlängert sein kann.

Überraschenderweise wurde festgestellt, daß die Deletion der übrigen, im nativen t-PA vorhandenen Bereiche keinen Einfluß auf die thrombolytische Wirksamkeit des Proteins hat und die fibrinabhängige Stimulierbarkeit des Muteins vergleichbar mit der von nativem t-PA ist. Zwar wurde festgestellt, daß dem erfindungsgemäßen t-PA-Derivat die Fibrin-Bindungseigenschaft fehlt, es jedoch überraschenderweise trotzdem eine in vivo Thrombolyse-Effizienz aufweist, die gegenüber der von nativem t-PA sogar wesentlich verbessert ist. Ebenso überraschend ist die Tatsache, daß bei Gabe einer thrombolytisch ausreichend wirksamen Dosis des Derivats die systemische Fibrinolyse nahezu unbeeinflußt bleibt. Es hat sich also gezeigt, daß das erfindungsgemäße t-PA-Derivat die für natives t-PA typische Eigenschaft der Fibrin-Spezifität unter physiologischen Bedingungen aufweist. Diese Ergebnisse wurden aus pharmakologischen Untersuchungen des erfindungsgemäßen t-PA-Derivats erhalten (siehe Beispiele 6 und 7). Außerdem weist das erfindungsgemäße Protein eine sehr hohe spezifische Aktivität auf. Bei Anwendung der beschriebenen Renaturierung wurden bereits Aktivitäten von 500 bis 800 kU/mg bestimmt.

Ein weiterer Gegenstand der Erfindung ist eine DNA-Sequenz, die für das erfindungsgemäße t-PA-Derivat kodiert und die folgende Sequenz enthält:

```
   1   ATGTCTTACCAAGGAAACAGTGACTGCTACTTTGGGAATGGGTCAGCCTACCGTGGCACG      60
  61   CACAGCCTCACCGAGTCGGGTGCCTCCTGCCTCCCGTGGAATTCCATGATCCTGATAGGC    120
 121   AAGGTTTACACAGCACAGAACCCCAGTGCCCAGGCACTGGGCCTGGGCAAACATAATTAC    180
 181   TGCCGGAATCCTGATGGGGATGCCAAGCCCTGGTGCCACGTGCTGAAGAACCGCAGGCTG    240
 241   ACGTGGGAGTACTGTGATGTGCCCTCCTGCTCCACCTGCGGCCTGAGACAGTACAGCCAG    300
 301   CCTCAGTTTCGCATCAAAGGAGGGCTCTTCGCCGACATCGCCTCCCACCCCTGGCAGGCT    360
 361   GCCATCTTTGCCAAGCACAGGAGGTCGCCCGGAGAGCGGTTCCTGTGCGGGGGCATACTC    420
 421   ATCAGCTCCTGCTGGATTCTCTCTGCCGCCCACTGCTTCCAGGAGAGGTTTCCGCCCCAC    480
 481   CACCTGACGGTGATCTTGGGCAGAACATACCGGGTGGTCCCTGGCGAGGAGGAGCAGAAA    540
 541   TTTGAAGTCGAAAAATACATTGTCCATAAGGAATTCGATGATGACACTTACGACAATGAC    600
 601   ATTGCGCTGCTGCAGCTGAAATCGGATTCGTCCCGCTGTGCCCAGGAGAGCAGCGTCGTC    660
 661   CGCACTGTGTGCCTTCCCCCGGCGGACCTGCAGCTGCCGGACTGGACGGAGTGTGAGCTC    720
 721   TCCGGCTACGGCAAGCATGAGGCCTTGTCTCCTTTCTATTCGGAGCGGCTGAAGGAGGCT    780
 781   CATGTCAGACTGTACCCATCCAGCCGCTGCACATCACAACATTTACTTAACAGAACAGTC    840
 841   ACCGACAACATGCTGTGTGCTGGAGACACTCGGAGCGGCGGGCCCCAGGCAAACTTGCAC    900
 901   GACGCCTGCCAGGGCGATTCGGGAGGCCCCCTGGTGTGTCTGAACGATGGCCGCATGACT    960
 961   TTGGTGGGCATCATCAGCTGGGGCCTGGGCTGTGGACAGAAGGATGTCCCGGGTGTGTAC   1020
1021   ACAAAGGTTACCAACTACCTAGACTGGATTCGTGACAACATGCGACCG   1068
```

Die erfindungsgemäße DNA-Sequenz dient zur Expression des erfindungsgemäßen t-PA-Derivats, wenn sie auf einem Expressionsplasmid vorhanden ist. Ein derartiges Expressionsplasmid ist ein weiterer Gegenstand der Erfindung, ebenso wie ein Expressionsplasmid, das eine hiervon abweichende DNA-Sequenz aufweist, die jedoch ebenfalls für das erfindungsgemäße t-PA-Derivat kodiert. Aufgrund der Degeneration des genetischen Codes sind hierfür von der gezeigten DNA-Sequenz abweichende Sequenzen geeignet.

Das Expressionsplasmid enthält vorzugsweise neben der für das t-PA-Derivat kodierenden Sequenz zusätzlich noch eine regulierbare Promotorstruktur (z.B. tac), einen effizienten Terminator (z.B. fd), einen Selektionsmarker (z.B. $\beta$-Lactamase-Gen) und einen origin of replication.

Ein weiterer Gegenstand der Erfindung ist das Plasmid pA27.3. Die Herstellung dieses Plasmids ist im Beispiel 1 beschrieben, es enthält eine DNA-Sequenz, die für das erfindungsgemäße t-PA-Derivat kodiert.

Wiederum ein weiterer Gegenstand der Erfindung ist ein Verfahren zur Herstellung eines der erfindungsgemäßen Expressionsplasmide, das dadurch gekennzeichnet ist, daS man eine DNA-Sequenz, die für das erfindungsgemäße t-PA-Protein oder ein Derivat davon, welches über die Kringel II- und die Protease-Domänen hinaus noch weitere Bereiche des t-PA-Proteins aufweist, kodiert, in ein Plasmid einbringt und über gerichtete Mutagenese diejenigen Bereiche entfernt, die für Aminosäuren kodieren, die im erfindungsgemäßen t-PA-Derivat nicht vorhanden sind.

Die Auswahl des Plasmids, in das die für das erfindungsgemäße t-PA-Derivat kodierende DNA-Sequenz eingebracht wird, ist abhängig von den später zur Expression des Derivats verwendeten Wirtszellen. Geeignete Plasmide sowie die Minimalanforderungen, die an ein derartiges Plasmid gestellt werden (z.B. Replikationsursprung, Restriktionsschnittstelle) sind dem Fachmann bekannt. Im Rahmen der Erfindung können jedoch anstelle eines Plasmids auch ein Cosmid, die replikative, doppelsträngige Form von Phagen (λ, M13), und andere dem Fachmann bekannte Vektoren verwendet werden. Die Methode der gerichteten Mutagenese (site-directed mutagenesis) ist von Morinaga et al., Biotechnology 21, (1984), 634, beschrieben, und wird im wesentlichen wie dort beschrieben ausgeführt.

Wiederum ein weiterer Gegenstand der Erfindung ist ein Verfahren zur Herstellung eines erfindungsgemäßen t-PA-Derivats, das dadurch gekennzeichnet ist, daß man eines der erfindungsgemäßen Plasmide in geeigneten Wirtszellen exprimiert und das Produkt aus dem Kulturmedium, gegebenenfalls nach Aufschluß der Wirtszellen gewinnt. Vorzugsweise werden zur Herstellung des erfindungsgemäßen t-PA-Derivats als Wirtszellen Prokaryontenzellen verwendet. Hierbei ist es wiederum besonders bevorzugt, daß man die sich hierbei bildenden sogenannten "inclusion bodies" (unlösliche Proteinaggregate) zunächst von den löslichen Zellpartikeln abtrennt, die t-PA enthaltenden inclusion bodies durch Behandlung mit Guanidin-Hydrochlorid unter reduzierenden Bedingungen solubilisiert, sie anschließend mit GSSG derivatisiert und zuletzt das t-PA-Derivat durch Zugabe von L-Arginin und GSH renaturiert. Genaue Vorschriften zur Aktivierung von t-PA aus "inclusion bodies" sind beispielsweise in EP-A 0 219 874 und EP-A 0 241 022 beschrieben. Jegliche andere Verfahren zur Gewinnung des aktiven Proteins aus "inclusion bodies" können jedoch erfindungsgemäß ebenso eingesetzt werden.

Bei dem erfindungsgemäßen Verfahren zur Aufreinigung von K2P arbeitet man bevorzugt in Anwesenheit von L-Arginin, insbesondere in einer Konzentration von 10 bis 1000 mmol/l.

Die erfindungsgemäße Abtrennung von Fremdprotein durch Affinitätschromatographie wird in einer bevorzugten Ausführungsform der Erfindung über eine ETI (Erythrina Trypsin Inhibitor) Adsorbersäule durchgeführt. Hierbei ist ETI auf einem Trägermaterial (Adsorber), wie z.B. Sepharose, fixiert. Die Reinigung über eine ETI-Adsorbersäule hat den Vorteil, daß das ETI-Adsorber-Säulenmaterial direkt aus dem konzentrierten Renaturierungsansatz sogar in Gegenwart von so hohen Arginin-Konzentrationen wie 0,8 mol/l Arginin, beladen werden kann. Eine Aggregation von K2P, wie sie bei niedrigen Arginin-Konzentrationen unter 10 mmol/l stattfinden kann, wird dadurch vermieden. Besonders bevorzugt erfolgt daher die Reinigung von K2P über eine ETI-Adsorbersäule in Gegenwart von 0,6 bis 0,8 Mol/l Arginin. Die K2P enthaltende Lösung hat dabei bevorzugt einen pH von über 7, besonders bevorzugt von 7,5 bis 8,6.

Die Elution von der ETI-Säule erfolgt durch pH-Erniedrigung, sowohl in Gegenwart als auch in Abwesenheit von Arginin unter Bedingungen, die eine gute Löslichkeit von K2P ermöglichen. Vorzugsweise liegt der pH-Wert bei der Elution im sauren Bereich, besonders bevorzugt im Bereich von 3 bis 5,5.

Ein erfindungsgemäß hergestelltes K2P besitzt eine spezifische t-PA-Aktivität von 550.000 ± 200.000 IU/mg bei einer Reinheit von über 95 %, vorzugsweise von über 99 %.

Erfindungsgemäß wird also ein t-PA-Derivat bereitgestellt, das eine deutlich verlängerte Plasma-Halbwertszeit aufweist aufgrund von reduzierter Clearance-Rate. Das erfindungsgemäße Derivat verliert jedoch hierdurch keine seiner es als Arzneimittel zur Thrombolyse arterieller und venöser Gerinnsel geeignet erscheinen lassenden Eigenschaften. Vielmehr kann die für eine thrombolytische Therapie mit K2P erforderliche Dosis auf mindestens ein Viertel der bei nativem t-PA üblichen Dosis reduziert werden. Bei gleichwirksamen Dosen von K2P und nativem t-PA wird das Gerinnungssystem durch K2P geringer beeinflußt als durch natives t-PA, und die Blutungszeit nicht signifikant verlängert im Unterschied zu nativem t-PA, so daß die Blutungskomplikationen bei der Therapie mit K2P möglicherweise vermindert werden können.

Das erfindungsgemäße t-PA-Derivat ist daher besonders zur Verwendung in einem Arzneimittel geeignet, was wiederum ein weiterer Gegenstand der Erfindung ist. Bei Verwendung des erfindungsgemäßen t-PA-Derivats in Arzneimitteln wird zur Erzielung von gleicher Wirksamkeit nur noch eine deutlich geringere Verabreichungsdosis benötigt als es bei Verwendung von nativem, in CHO produziertem t-PA der Fall ist.

Die folgenden Beispiele erläutern die Erfindung in Verbindung mit den Abbildungen weiter.

Fig. 1      zeigt schematisch die Herstellung des Plasmids pA27.3;

Fig. 2      zeigt den Vergleich der Fibrinbindung des erfindungsgemäßen t-PA-Derivats (Kurve 1) mit in CHO-Zellen exprimiertem nativen t-PA (zweikettiger t-PA aus CHO-Zellen, gespalten an der physiologischen Spaltungsstelle Arg 275-Ile276, Kurve 2) und einkettigem t-PA aus CHO-Zellen, (Kurve 3)

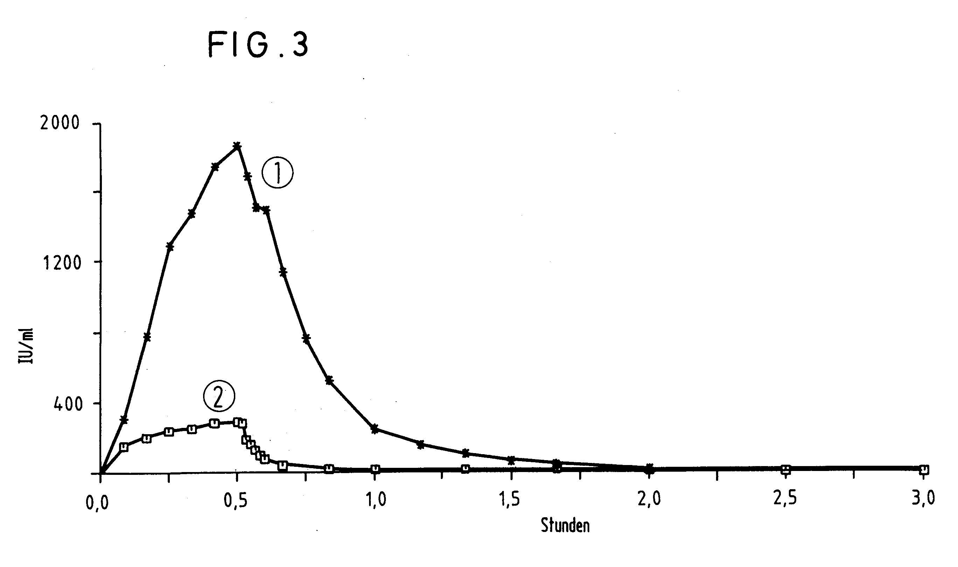

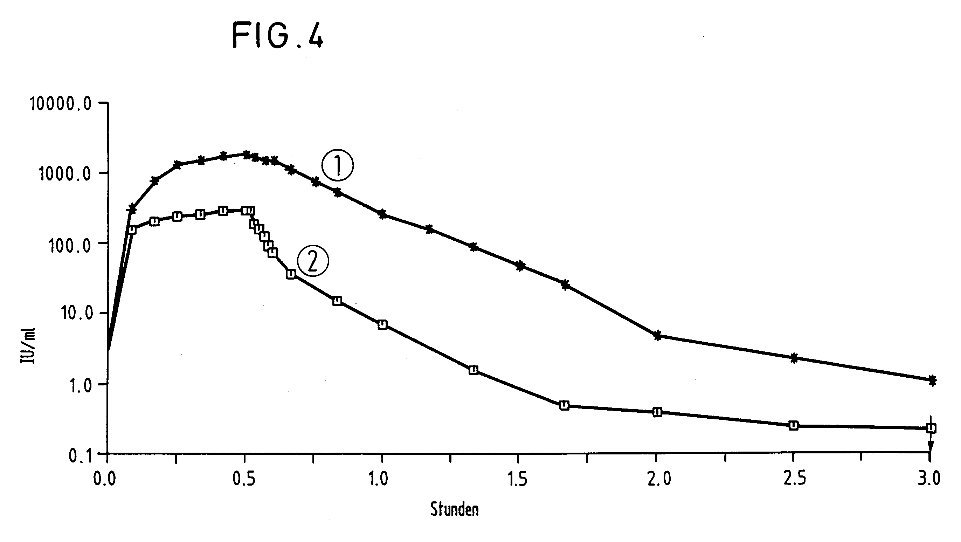

Fig. 3 und Fig. 4    zeigen Diagramme zur Pharmakokinetik der t-PA-Aktivität des erfindungsgemäßen t-PA-Derivats, verglichen mit einem kommerziell erhältlichen t-PA-Präparat (Actilyse®); (Kurve 1: K2P, Dosis 200.000 U/kg = 0,25 mg/kg; i.v. Inf. über 30 min.; Anzahl der untersuchten Tiere (Kaninchen): 4; Kurve 2: Actilyse®, Dosis 200.000 U/kg; i.v. Inf. über 30 min., Anzahl der untersuchten Tiere (Kaninchen): 6).

Fig. 5    zeigt Dosiswirkungskurven (für Kaninchen) der Thrombolyse des erfindungsgemäßen t-PA-Derivats, verglichen mit Actilyse® (gezeigt ist der Mittelwert + SEM, 1 kU ≙ 1000 IU; Kurve 1: K2P; Kurve 2: Actilyse®).

Fig. 6    zeigt den zeitlichen Verlauf der Simplate Blutungszeit (BT) vor und nach i.v. Bolusinjektion von Placebo oder steigender Dosen von Actilyse® in narkotisierten Hunden.

Fig. 7    zeigt den zeitlichen Verlauf der Simplate Blutungszeit (BT) vor und nach i.v. Bolusinjektion von Placebo oder steigender Dosen von K2P in narkotisierten Hunden.

**Beispiel 1**

Konstruktion des Plasmids pA27.3

Das Ausgangsplasmid pREM7685, beschrieben in EP-A 0 242 836, enthält folgende Komponenten: tac-Promotor, lac-Operator-Region mit einem ATG-Startcodon, die kodierende Region für das t-PA-Derivat FK2P, den Transkriptionsterminator aus pKK223-3, ein ß-Lactamase-Gen, ein Kanamycin-Resistenz-Gen und den Origin des Plasmids pACYC177, eines Plasmids, das in der Zelle in niedriger Kopienzahl vorliegt. Die Sequenz des t-PA-Derivats FK2P setzt sich zusammen aus den Nukleotiden 190-336 (F-Domäne), 715-1809 (K2-Domäne,Protease, geringer Anteil 3'UT) und einem ATG-Startcodon. Die Nukleotidpositionen werden gemäß der von Pennica et al., Nature 301 (1983) 214-221, beschriebenen Sequenz angegeben.

Zur Deletion der F-Domäne aus der FK2P-Konstruktion in Plasmid pREM7685 wurde im wesentlichen die Methode von Morinaga et al., Biotechnology 21 (1984), 634, angewendet. Zur Heteroduplexbildung wurden aus pREM7685 zwei Fragmente isoliert. Fragment A: pREM7685 wurde mit dem Restriktionsenzym EcoRI gespalten. Die Spaltprodukte wurden gelelektrophoretisch aufgetrennt und das größte EcoRI-Fragment aus dem Gel eluiert. Fragment B: Plasmid pREM7685 wurde mit dem Restriktionsenzym XhoI linearisiert. Das linearisierte Plasmid wurde ebenfalls nach Gelelektrophorese präparativ erhalten. Für die Mutagenese wurde folgendes Oligonukleotid synthetisch hergestellt.

5' TG TCT TAC CAA GGA AAC AGT GA 3'

Zur Heteroduplex-Bildung wurden Fragment A, Fragment B (je 450 fmol) und das Oligonukleotid (75 pMol) gemischt und in Gegenwart von 50 mmol/l NaCl, 10 mmol/l Tris-HCl, pH 7,5 und 10 mmol/l MgSO₄ zunächst drei Minuten bei 100°C inkubiert und sofort auf Eis überführt. Die Renaturierung der DNA erfolgte für 30 Minuten bei 60°C. Zur Reparatursynthese wurde dem Heteroduplex folgendes hinzugefügt: Desoxynukleotidtriphosphate (0,25 mmol/l), ATP (1 mmol/l), NaCl (100 mmol/l), Tris-HCl, pH 7,5 (6,5 mmol/l), MgCl₂ (8 mmol/l), ß-Mercaptoethanol (1 mmol/l), Klenow-Fragment der DNA-Polymerase aus E. coli (0,125 U/µl Ansatz) und T4-Ligase (0,1 U/µl Ansatz). Die Reparatursynthese erfolgte für 4 Stunden bei 16°C. Anschließend wurde dieser Ansatz in E. coli-Zellen (RM82, DSM 3689) mit einem lac Iq-Plasmid transformiert und die Transformanten durch Zugabe von 25 µg/ml Kanamycin zum Nährmedium selektioniert.

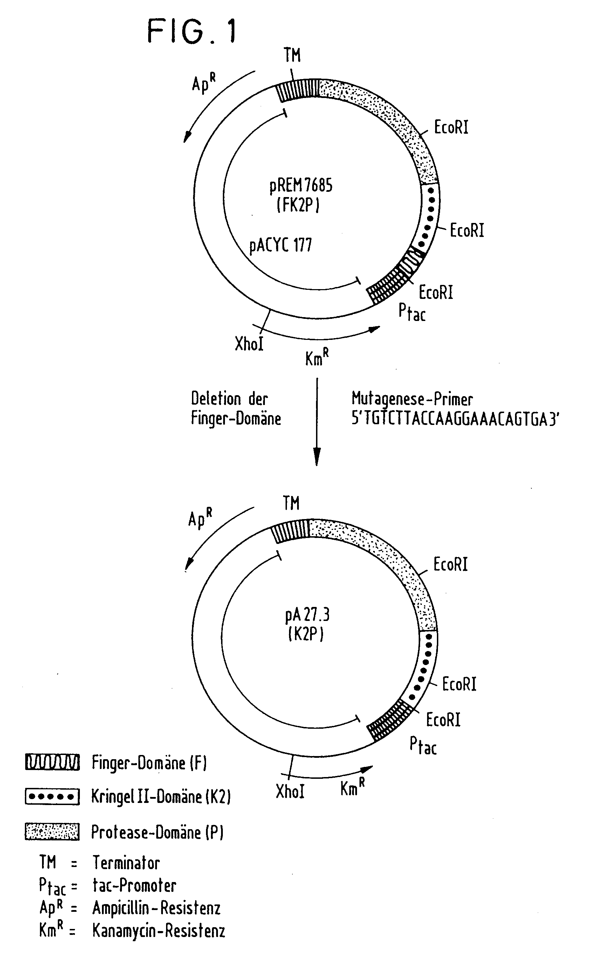

Mit Hilfe der Koloniehybridisierungs-Technik unter Verwendung des oben beschriebenen Mutagenese-Oligonukleotids als Sonde konnten jene Klone ausgewählt werden, die das Plasmid pA27.3 tragen, welches das erfindungsgemäße t-PA-Derivat K2P kodiert. Dieses Plasmid unterscheidet sich vom Ausgangsplasmid pREM7685 u. a. durch das Fehlen einer PstI- bzw. einer SspI-Schnittstelle. Diese beiden Schnittstellen sind in jenem Bereich des Ausgangsplasmids enthalten, der für die F-Domäne kodiert. Die Konstruktion des Plasmids pA27.3 ist schematisch in Fig. 1 dargestellt.

**Beispiel 2**

Präparation von aktivem t-PA-Derivat K2P aus E. coli

Zellyse und Präparation der inclusion bodies (IB's)

1,6 kg Zellfeuchtmasse (E. coli, DSM 3689, transformiert mit Plasmid pA27.3) wurden in 10 l 0,1 mol/l Tris-HCl, 20 mmol/l EDTA, pH 6,5, 4°C suspendiert. Dazu wurde 2,5 g Lysozym zugegeben und 30 Minuten bei 4°C inkubiert; anschließend wurde vollständiger Zellaufschluß mittels Hochdruckdispersion durchgeführt. Zur Aufschlußlösung wurden 5 l 0,1 mol/l Tris-HCl, 20 mmol/l EDTA, 6 % Triton X100 und 1,5 mol/l NaCl, pH 6,5 zugemischt und weitere 30 Minuten bei 4°C inkubiert. Daran anschließend folgte die Abtrennung der unlöslichen Bestandteile (IB's) durch Zentrifugation mit einer Padberg-Zentrifuge.

Das Pellet wurde in 10 l 0,1 mol/l Tris-HCl, 20 mmol/l EDTA, pH 6,5 suspendiert, 30 Minuten bei 4°C inkubiert und das IB-Präparat durch anschließende Zentrifugation isoliert.

Solubilisierung der IB's

100 g IB's (Naßgewicht) wurden in 450 ml 0,1 mol/l Tris-HCl, 6 mol/l Guanidin•HCl, 0,2 mol/l DTE (1,4 Dithioerythrit), 1 mmol/l EDTA, pH 8,6 suspendiert und 2,5 Stunden bei 25°C gerührt.

Nach Einstellen des pH-Wertes auf pH 3 mit HCl (25 %) wurde eine Dialyse gegen 10 mmol/l HCl (3 x 50 l, 24 Stunden, 4°C) durchgeführt.

Derivatisierung

Guanidin•HCl (fest) wurde vorgelegt, so daß nach Endverdünnung des obengenannten Dialysats mit 10 mmol/l HCl die Konzentration von Guanidin-HCl 6 mol/l betrug.

Der Ansatz wurde bei 25°C 1,5 Stunden vorinkubiert, anschließend wurde oxidiertes Glutathion (GSSG) ad 0,1 mol/l und Tris-HCl ad 0,05 mol/l eingestellt und der pH-Wert mit 5 mol/l NaOH auf pH 9,3 titriert. Der Ansatz wurde 3,5 Stunden bei 25°C gerührt.

Nach Einstellung des pH-Wertes auf pH 3 mit HCl (25 %) wurde eine Dialyse gegen 10 mmol/l HCl (3 x 100 l, 48 Stunden, 4°C) durchgeführt. Nach der Dialyse wurde zentrifugiert und der klare Überstand weiterverarbeitet.

Naturierung

Ein 10 l Reaktionsgefäß wurde mit 0,1 mol/l Tris-HCl, 0,8 mol/l L-Arginin, 2 mmol/l GSH (Genthation, (reduzierte Form)), 1 mmol/l EDTA, pH 8,5 gefüllt. Die Renaturierung wurde bei 20°C durch dreifache Zugabe von jeweils 100 ml Derivat (gemischtes Disulfid, s. o.) im Zeitabstand von 24 Stunden bewirkt.

Nach der Renaturierung erhält man ein Material mit einer spezifischen Aktivität von 1500 bis 10.000 IU/mg (Bestimmung vgl. Beispiel 4b). Die Einheit IU ist eine Einheit der Aktivität nach Definition der WHO, National Institute for Biological Standards and Control.

Konzentrierung des Renaturierungsansatzes

Der Renaturierungsansatz kann bei Bedarf über einen Hemodialysator konzentriert werden.

**Beispiel 3**

Reinigung von K2P aus E. coli

1. Reinigung von K2P aus E. coli über Affinitätschromatographie an ETI-Sepharose nach vorheriger Konzentration
   a) Elution mit Citronensäure
   Der Renaturierungsansatz wurde über einen Hemodialysator (Asahi AM 300) 1:23 konzentriert und mit 0,5 mol/l NaCl aufgestockt. Eine mit 0,1 mol/l Tris-HCl, pH 7,5, 0,8 mol/l Arginin, 0,5 mol/l NaCl äquilibrierte ETI(Erythrina-Trypsin-Inhibitor)-Sepharose-Säule (V = 50 ml) wurde mit 550 ml Konzentrat des Reoxidationsansatzes beladen (10 Säulenvolumen/h, 10 SV/h) und so lange mit dem Äquilibrierungspuffer gewaschen, bis die Absorption des Eluats bei 280 nm den Leerwert des Puffers erreichte. Die Elution des gebundenen Materials erfolgte mit 20 mmol/l Citronensäure, pH 3,2.

|  | Volumen ml | Aktivität IU/ml | $C_{Prot.}$ mg/ml | SA [1] IU/mg |
|---|---|---|---|---|
| Konzentrat | 550 | 57.162 | 14 | 4.083 |
| ETI-Eluat | 90 | 330.000 | 0,71 | 465.000 |

[1] Spezifische Aktivität; Aktivität im chromogenen Test (vgl. Beispiel 4b) geteilt durch Proteingehalt der Probe

b) Elution mit 0,3 Mol/l Arg, pH 4,5

Der Renaturierungsansatz wurde wie in Beispiel 3.1.a) beschrieben, konzentriert. Eine mit 0,1 mol/l Tris-HCl, pH 7,5, 0,8 mol/l Arginin, 0,5 mol/l NaCl äquilibrierte ETI-Sepharose-Säule (25 ml) wurde mit 800 ml Konzentrat beladen (12 SV/h) und so lange mit dem Äquilibrierungspuffer gewaschen, bis die Extinktion des Eluats bei 280 nm die Extinktion des Puffers erreichte. Das gebundene Material wurde mit 0,3 mol/l Arginin, pH 4,5 eluiert.

|  | Volumen ml | Aktivität IU/ml | $C_{Prot.}$ mg/ml | SA IU/mg |
|---|---|---|---|---|
| Konzentrat | 800 | 20.000 | 11,3 | 1770 |
| ETI-Eluat | 55 | 280.000 | 0,6 | 550.000 |

2. Reinigung von K2P aus E. coli über Affinitätschromatographie an ETI-Sepharose ohne vorherige Konzentration

Eine mit 0,1 mol/l Tris-HCl, pH 7,5, 0,8 mol/l Arginin, 0,5 mol/l NaCl äquilibrierte ETI-Sepharose-Säule (V = 10 ml) wurde mit 12 l Reoxidationsansatz beladen und so lange mit dem Äquilibrierungspuffer gewaschen, bis die Extinktion des Eluats bei 280 nm die Extinktion des Puffers erreichte. Die Elution des gebundenen Materials erfolgte mit 0,8 mol/l Arginin, pH 5.

|  | Volumen ml | Aktivität IU/ml | $C_{Prot.}$ mg/ml | SA IU/mg | F[1] |
|---|---|---|---|---|---|
| Reoxidationsansatz | 12.000 | 615 | 0,135 | 4556 | 25 |
| ETI-Eluat | 42 | 105.000 | 0,185 | 568.000 | 35 |

[1] F: Stimulation durch Fibrin = Aktivität in Gegenwart von Fibrin geteilt durch Aktivität ohne Fibrin

**Beispiel 4**

Charakterisierung von gereinigtem K2P aus E. coli

a) Proteinchemische Charakterisierung

- SDS-Page und Reverse Phase HPLC

Die Homogenität des über Affinitätschromatographie an ETI-Sepharose gereinigten Materials wurde durch SDS-PAGE und durch Reverse Phase HPLC (RP-HPLC) gezeigt. Aus der relativen Laufstrecke errechnet sich für K2P aus Prokaryonten ein Molekulargewicht von 38.500 ± 2.000 Da. Die densitometrische Auswertung ergab eine Reinheit des Präparats von >95 %.

Die RP-HPLC beruht auf der unterschiedlichen Wechselwirkung von Proteinen mit hydrophoben Matrices. Diese Eigenschaft wurde als analytische Methode zur Quantifizierung des Reinheitsgrades angewandt.

Die Analyse des gereinigten K2P aus E. coli erfolgte über eine Nucleosil 300-Trennsäule (Knauer) mit Hilfe eines Trifluoressigsäure/Acetonitril-Gradienten (Puffer A: 1,2 ml Trifluoressigsäure in 1000 ml $H_2O$; Puffer B: 300 ml $H_2O$, 700 ml Acetonitril, 1 ml Trifluoressigsäure; 0 bis 100 %). Die Integration der chromatographischen Analyse ergab eine Reinheit von >95 %.

- N-terminale Aminosäuresequenz

Die N-terminale Aminosäuresequenz wurde mit einem ABI 470 Sequenator mit Standardprogramm und on line PTH Detektion bestimmt. Die gefundene Sequenz S1-Y2-Q3-G4-N5-S6-D7-C8-Y9 stimmt mit der aus der DNA-Sequenz abgeleiteten, zu erwartenden Sequenz überein.

b) Aktivitätsbestimmung

Die in vitro-Aktivität von K2P aus E. coli wurde nach der Testvorschrift in "Zeitschrift für die gesamte innere Medizin" (ZGIMAL) 42 (17) 478-486 (1987) bestimmt. Die spezifische Aktivität betrug 550.000 IU/mg ± 200.000 IU/mg. Die Stimulierbarkeit von K2P aus E. coli durch BrCN-Fragmente des Fibrinogens (Aktivität in Gegenwart von Fibrinogen-Fragmenten geteilt durch Aktivität ohne Fibrinogenfragmente) war in diesem Testsystem >25.

c) In vitro-Bindung an Fibrin

Die in vitro-Bindung an K2P aus E. coli, an Fibrin wurde nach der von Higgins und Vehar beschriebenen Methode bestimmt (Higgins, D.L. und Vehar, G.A. (1987), Biochem. 26, 7786-7791).

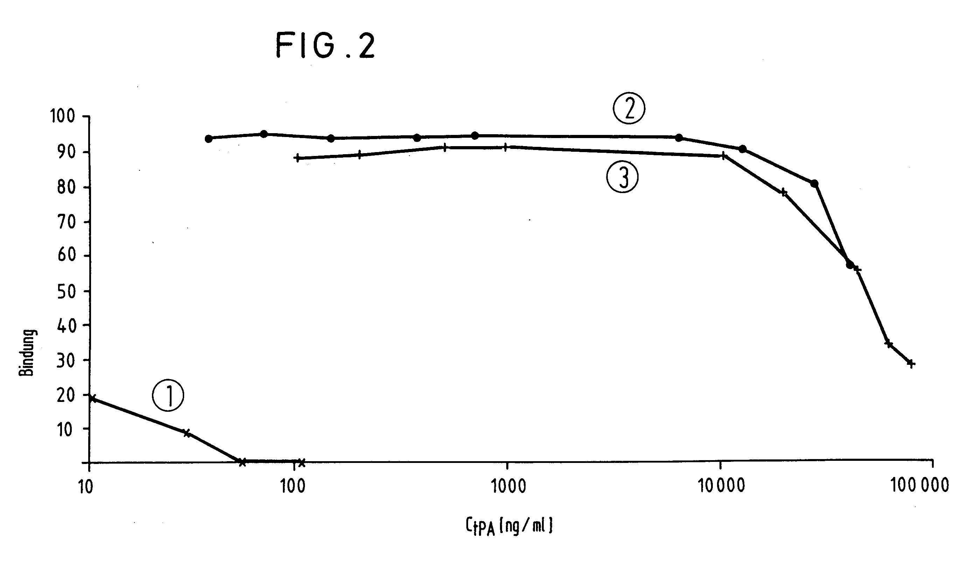

Abbildung 2 zeigt, daß K2P aus E. coli im Gegensatz zu t-PA aus CHO oder t-PA aus E. coli keine nennenswerte Fibrinbindung aufweist.

**Beispiel 5**

Zur Steigerung der Expressionsausbeute wurde die für das K2P-Gen kodierende Sequenz in ein Plasmid hoher Kopienzahl umkloniert. Dazu wurde das in der Patentanmeldung DE 38 38 378.0 beschriebene Plasmid pePa126.1 benutzt. Dieses Plasmid setzt sich im wesentlichen aus dem Vektor pKK223-3 und der kodierenden Sequenz für t-PA zusammen, wie sie in EP-A 0 242 835 beschrieben ist.

In dieses Plasmid wurde zunächst die Sequenz eines fd-Terminators integriert. Dazu wurde das Plasmid pePa 126.1 mit dem Restriktionsenzym Hind III linearisiert. Das so gespaltene Plasmid wurde gelelektrophoretisch aufgetrennt und präparativ gewonnen. Das Plasmid pLBUI (Beck et al. (1978), Nucl.Acids Res., 5, 4495-4503; Gentz et al. (1981) PNAS 78(8):4963) wurde mit Hind III restringiert und ein etwa 360 bp großes Hind III-Fragment, das den fd-Terminator enthält, durch Gelelektrophorese und Gel-Elution präparativ dargestellt. Das linearisierte Plasmid pePa126.1 und das 360 bp Hind III-Fragment aus pLBUI wurde ligiert. Der Ligationsansatz wurde mit dem in der Anmeldung DE 38 38 378.0 beschriebenen Plasmid pUBS500 in E. coli, DSM 2102, kotransformiert. Aus den Klonen wurden diejenigen ausgewählt, die das gewünschte Plasmid pePa126fd enthalten, das sich vom Ausgangsplasmid pePa126.1 dadurch unterscheidet, daß es eine zweite Hind III-Schnittstelle enthält.

Aus dem Plasmid pePa126fd wurden zwei Fragmente gewonnen: ein 3,4 kb großes BamHI/PvuI-Fragment und ein etwa 1,3 kb großes PvuI/XmaI-Fragment. Die beiden genannten Fragmente wurden mit einem etwa 1,3 kb großen BamHI/XmaI-Fragment aus dem Plasmid pA27.3 ligiert und mit dem Plasmid pUBS500 in E. coli transformiert. Das resultierende Plasmid erhielt den Namen pA27 fd und kann von pePa126fd dadurch unterschieden werden, daß bei einem Restriktionsansatz mit EcoRI das zweitkleinste EcoRI-Fragment aus pePa126fd von etwa 610 bp Länge in pA27fd um etwa 515 bp verkürzt vorliegt.

**Beispiel 6**

Pharmakologische Befunde von in Prokaryonten exprimiertem t-PA-Derivat K2P

1. Pharmakokinetik von K2P am Kaninchen

K2P wurde an weißen Neuseeland-Kaninchen mit Actilyse® in seinen pharmakokinetischen Eigenschaften verglichen. Beide Fibrinolytika wurden in einer Dosis von 200.000 IU/kg KG 30 Minuten lang infundiert. Plasmaproben wurden zu definierten Zeitpunkten vor, während und nach der Infusion gewonnen. Die t-PA-Aktivität wurde mit einem spektrophotometrischen Test nach J. H. Verheijen et al. (Thromb. Haemostas. 48, 266, 1982), modifiziert nach H. Lill (Z. ges. Inn. Med. 42, 478, 1987), gemessen.

Zur Berechnung pharmakokinetischer Parameter wurde ein Rechenprogramm der nichtlinearen Regression, modifiziert nach H. Y. Huang (Aero-Astronautics-Report 64, Rice University, 1-30, 1969) benutzt. Die Parameter wurden individuell mit Hilfe eines bi-exponentiellen pharmakokinetischen Modells berechnet.

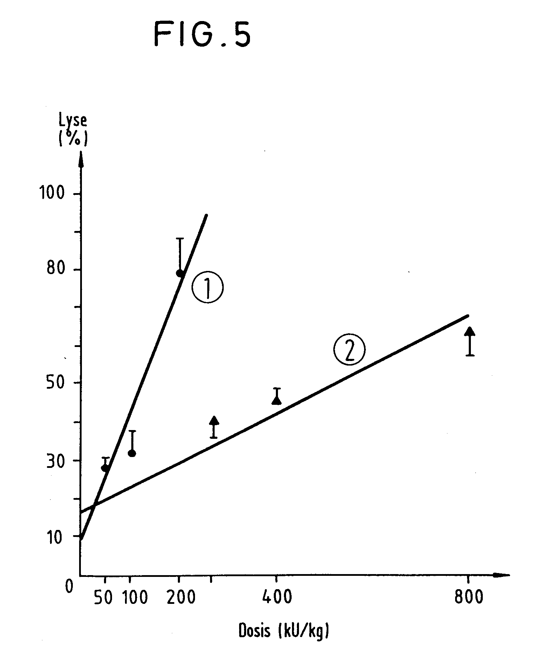

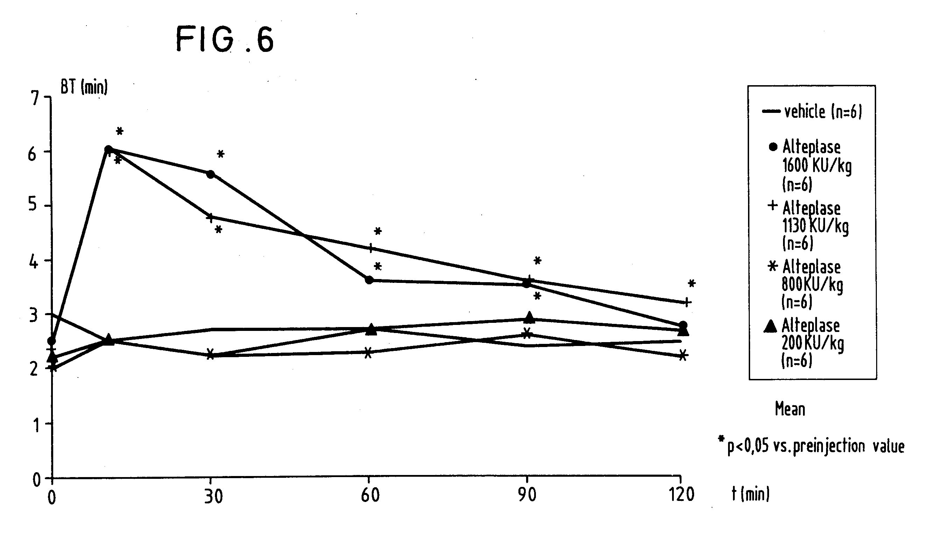

K2P weist eine fünffach längere Halbwertszeit ($t1/2\alpha$ = 10,3 min, Abnahme der Konzentration in Plasma) als Actilyse® (t-PA- Präparat der Firma Thomae) auf (Tabelle 1, Abbildung 5 und 6). Am Ende der Infusion (nach 30 min) wurde bei K2P eine Plasmakonzentration der t-PA-Aktivität ($C_{inf}$) von 1986 IU/ml gemessen, die somit sechsmal hoher als bei Actilyse® war. Das Verteilungsvolumen des zentralen Kompartiments ($V_c$) betrug für K2P 46,8 ml/kg im Vergleich zu 73,7 ml/kg bei Actilyse®. Die Gesamtplasmaclearance ($Cl_{tot}$) war gegenüber Actilyse® ($Cl_{tot}$ = 22,2 ml/min/kg) bei K2P auf 1/7 reduziert ($Cl_{tot}$ = 3,2 ml/min/kg). Für die Anwendung eines Fibrinolytikums zur Bolusinjektion ist die "Area under the curve" (AUC) besonders interessant, da sie einen Vergleich der über die Zeit im Plasma herrschenden Konzentration zuläßt. K2P zeigte eine achtfach höhere AUC (1064 IU/ml•h) als Actilyse® (133,3 IU/ml•h).

Insgesamt zeigte K2P im Vergleich mit Actilyse®, dem derzeitig einzigen kommerziell erhältlichen rekombinanten t-PA-Protein, bei gleicher Dosis ein fünf- bis achtfach verbessertes pharmakokinetisches Profil.

2. Pharmakodynamik von K2P am Kaninchen

Zur Prüfung der thrombolytischen Effizienz wurde das von D. Collen et al. etablierte Kaninchenmodell der Jugularvenenthromose angewandt (J. Clin. Invest. 71, 368, 1983). K2P und Actilyse® wurden in jeweils 3 Dosen untersucht. Die Fibrinolytika wurden 4 Stunden lang infundiert und anschließend die Thrombolyserate ermittelt (Tabelle 2, Abbildung 5).

Die mit Hilfe einer linearen Regressionsgeraden errechnete Dosis für eine Thrombolyserate von 50 % ($ED_{50}$) lag für K2P bei 124.000 IU/kg KG und für Actilyse® bei 520.000 IU/kg KG. K2P zeigte somit eine vierfach höhere thrombolytische Wirkung als Actilyse.

K2P erzielte dosisabhängig eine Plasmakonzentration der t-PA-Aktivität, die bei vierfach niedrigerer Dosis vergleichbar war mit Actilyse®. Die in der thrombolytischen Wirkung mit 800 kU Actilyse®/kg KG vergleichbare Dosis von 200 kU K2P/kg KG führte zu geringen Auswirkungen auf die Gerinnungsparameter Fibrinogen, Plasminogen und $\alpha_2$-Antiplasmin, die sich jedoch nicht von den Auswirkungen einer Dosis von 800 kU Actilyse®/kg KG unterscheiden.

K2P ist ein t-PA-Mutein, das am Jugularvenenthrombose-Modell des Kaninchens bei vierstündiger Infusion der Fibrinolytika bei einer Dosisreduktion auf 1/4 der Actilyse®-Dosis die gleiche thrombolytische Wirkung wie Actilyse® entfaltet. K2P unterscheidet sich bei der reduzierten Dosis nicht von Actilyse® in den Wirkungen auf das Gerinnungssystem und in der Plasmakonzentration der t-PA-Aktivität.

## Tabelle 1

Pharmakokinetische Parameter, abgeleitet aus Computerberechnungen von t-PA-Plasmakonzentrations – Zeit Daten auf Basis der t-PA-Aktivität

| Substanz<br><br>(Dosis: 200 000 IU/kg KG) | $t_{1/2\alpha}$<br><br>(min) | $t_{1/2\beta}$<br><br>(min) | $C_{inf}$<br><br>(IU/ml) | $V_C$<br><br>(ml/kg) | $Cl_{tot}$<br><br>(ml/min/kg) | $AUC_{extrapol.}$<br><br>$(\frac{IU}{ml}\cdot h)$ |
|---|---|---|---|---|---|---|
| K2P<br>(n = 4) | 10,3<br>±1,7 | 14,9<br>±4,6 | 1986,6<br>±762,6 | 46,8<br>±14,7 | 3,2<br>±1,1 | 1064,4<br>±443,2 |
| Actilyse[(R)]<br>(n = 6) | 2,1<br>±0,6 | 10,9<br>±2,4 | 326,6<br>±118,1 | 73,7<br>±19,7 | 22,2<br>±7,6 | 133,3<br>±44,1 |

EP 0 382 174 B1

Tabelle 2

Thrombolyse, t-PA-Plasma Aktivitätsspiegel (am Ende einer 4-Stunden-Infusion) und Hämostasis-Parameter (30 Minuten nach Beendigung der Infusion) von K2P, Actilyse(R) und Lösungsmittel

| | K2P 200 kU/kg | K2P 100 kU/kg | K2P 50 kU/kg | Lösungsmittel NaCl/Tween | Actilyse(R) 800 kU/kg | Actilyse(R) 400 kU/kg |
|---|---|---|---|---|---|---|
| Thrombolyse (%) | 79 ± 9 (n = 3) | 32 ± 6 (n = 5) | 29 ± 1 (n = 2) | 11 ± 1 (n = 6) | 64 ± 6 (n = 7) | 46 ± 3 (n = 6) |
| Plasma t-PA-Aktivität (IU/ml) | 93,7 ± 18,2 | 44 ± 3 | 7 ± 0 | – | 107 ± 27 | 47 ± 9 |
| Fibrinogen (%) | 74 ± 2 | 90 ± 6 | 86,5 ± 6 | 92 ± 3 | 77 ± 6 | 90 ± 3 |
| Plasminogen (%) | 79 ± 7 | 75 ± 6 | 87 ± 11 | 98 ± 10 | 77 ± 4 | 88 ± 4 |
| $\alpha_2$-Antiplasmin (%) | 70 ± 1 | 70 ± 4 | 93 ± 4 | 98 ± 8 | 74 ± 6 | 87 ± 3 |

Mittelwert ± SEM; kU = 1000 IU; Hämostasis-Parameter (% bezogen auf Grundlinie)

Beispiel 7

Pharmakologische Eigenschaften von K2P aus E. coli an einem Hundemodell der koronararterienthrombose

Um die thrombolytische Wirkung von K2P aus E. coli auf arterielle Thromben zu untersuchen, wurde beispielhaft ein tierexperimentelles Modell für den akuten Myokardinfarkt gewählt. Als Tierspezies wurde der Hund ausgesucht. Die Methode zur Bildung eines Koronararterienthrombus stellt eine Modifikation der Technik von Romson et al. (Thromb. Res. 17, 841, 1980) dar. Am geöffneten Brustkorb wird bei den narkotisierten und beatmeten Hunden die Intimaoberfläche des Ramus circumflexus der A. coronaria sinistra ( = left circumflex coronary artery = LCX) elektrisch gereizt (150 $\mu$A) und dadurch ein Thrombus erzeugt. Distal der Thrombose war zuvor eine Schraube angelegt worden, um durch die experimentelle Stenose die reaktive Hyperämie zu eliminieren. Proximal der Koronarthrombose war die LCX mit einem elektromagnetischen Flowmeßkopf instrumentiert worden, um die Reperfusion messen zu können.

In einer Dosisfindungsstudie wurde BM 06.022 mit dem eukaryotischen t-PA (Alteplase, Actilyse®, Dr. Karl Thomae GmbH, Biberach, FRG) in je vier verschiedenen Dosen und mit Placebo an jeweils 6 Tieren/Dosis als initialer einmaliger intravenöser Bolus über 1 min den heparinisierten Hunden injiziert. Vor und zu definierten Zeitpunkten nach der Injektion wurden Plasmaproben gewonnen, um die Plasmakonzentration der t-PA-Aktivität und von Fibrinogen, Plasminogen und $\alpha_2$-Antiplasmin sowie um die Thrombozytenzahl im Vollblut zu bestimmen. Fibrinogen wurden koagulometrisch nach Clauss (Acta haemat. 17, 237, 1957), Plasminogen und $\alpha_2$-Antiplasmin wie von Collen et al. beschrieben (J. Clin. Invest. 71, 368, 1983) spektrophotometrisch gemessen. Weiterhin wurde die "Simplate Blutungszeit" am Hinterbein der Hunde mit Hilfe eines Schnäppers (Simplate® I, Organon Teknika, Eppelheim, FRG) während eines venösen Staus von 40 mm Hg gemessen (J. Surg. Res. 27, 244, 1979). Der statistische Vergleich der Meßwerte nach Injektion mit dem Kontrollwert vor Injektion erfolgte mit dem Wilcoxon-Test für Paardifferenzen.

Um den thrombolytischen Erfolg beschreiben zu können, wurde die Anzahl der pro Dosis-Gruppe reperfundierten Tiere ( = Reperfusionsrate) sowie die Zeit bis zur Reperfusion ( = Reperfusionszeit) angegeben. Weiterhin wurde das Feuchtgewicht des 2 h nach Injektion noch vorhandenen Restthrombus gemessen, und die Anzahl der Tiere mit Wiederverschluß nach Reperfusion ( = Reocclusionsrate) ermittelt. Mit Hilfe einer halblogarithmischen Regressionsanalyse der Dosiswirkungs (Reperfusionsraten)-Beziehung wurde für jede Substanz die effektive Dosis für eine 50 %-Reperfusionsrate ( = $ED_{50}$) errechnet. Der statistische Vergleich der Restthrombusgewichte erfolgte mit dem Wilcoxon-Mann-Whitney-Test für unverbundene Stichproben.

Die Plasmakonzentration der t-PA-Aktivität wurde mit einem spektrophotometrischen Test nach Verheijen et al. (Thromb. Haemost. 48, 266., 1982), modifiziert nach Lill (Z. gesamte Inn. Med. 42, 478, 1987), gemessen. Zur Berechnung pharmakokinetischer Parameter wurde ein Rechenprogramm der nichtlinearen Regression, modifiziert nach H.Y. Huang (AeroAstronautics Report 64, Rice University, USA, 1-30, 1969) benutzt. Die Parameter wurden individuell nach Abzug des körpereigenen Basalspiegels der t-PA-Aktivität von den nachfolgenden Meßwerten mit Hilfe eines bi-exponentiellen pharmakokinetischen Modells berechnet.

Folgende Ergebnisse wurden erhalten:

## 1. Pharmakodynamik am Hund

K2P führte nach intravenöser Injektion zu einer dosisabhängigen Reperfusionsrate. Der maximale Effekt (Reperfusionsrate von 100 %) wurde nach Injektion von 200 kU/kg KG erzielt. Die Dosis mit 100 % Reperfusionserfolg bei Actilyse[R] betrug 1600 kU/kg KG. Ein Vergleich der $ED_{50}$-Werte ergab für K2P ($ED_{50}$ = 83 kU/kg KG) einen 11,5-fach niedrigeren Wert als für Actilyse® ($ED_{50}$ = 961 kU/kg KG). Die Verabreichung von Placebo führte zu keiner Reperfusion. Das Restthrombusgewicht der Placebo-Tiere betrug 9,6 ± 1,6 mg (Mittelwert ± SEM); sowohl K2P als auch Actilyse[R] führten mit steigenden Dosen zu einer statistisch signifikanten Erniedrigung des Restthrombusgewichts im Vergleich mit der Placebo-Kontrolle. Die Reperfusion erfolgte bei beiden Fibrinolytika im Durchschnitt aller reperfundierten Tiere nach 25,9 ± 3,5 min K2P bzw. nach 24,2 ± 6,2 min (Actilyse®). Die meisten der mit K2P oder Actilyse® behandelten Hunde reoccludierten nach Reperfusion (Tab. 3).

## 2. Pharmakokinetik am Hund

Nach intravenöser Injektion von 200 kU/kg K2P oder Actilyse® zeigte sich am narkotisierten Hund, daß die schnelle Phase der Abnahme der Plasmakonzentration, ausgedrückt als $t_{1/2\alpha}$, bei K2P mit 7,2 ± 1,1 min um den Faktor 4,5 länger ist als bei Actilyse® mit 1,6 ± 0,2 min (Tab. 4). Die unmittelbar nach Beendigung der Injektion gefundene Plasmakonzentration von K2P war etwa doppelt so hoch wie bei Actilyse[R]. Die Entfernung von K2P aus dem Plasma (Plasmaclearance = $Cl_{tot}$) erfolgte neunmal langsamer als bei Actilyse[R]. Entsprechend war die Fläche unter der Plasmakonzentrationszeitkurve von K2P ungefähr 9,5 mal größer als die von Actilyse®.

### 3. Fibrinspezifität am Hund

Zwei Stunden nach Injektion von K2P fand sich eine dosisabhängig geringgradige Erniedrigung der Restkonzentration von Fibrinogen auf 81 ± 10 % bei der höchsten Dosis (200 kU/kg KG). Demgegenüber war die Fibrinogenkonzentration nach Verabreichung der höchsten Dosis von Actilyse[R] (1600 kU/kg KG) fast völlig auf 3 ± 0 % reduziert (Tab. 5). Führt man eine halblogarithmische Regressionsanalyse der Dosis-Nebenwirkung (Fibrinogenreduktions)-Beziehung durch und ermittelt die zur $ED_{50}$ thrombolytischen Wirkung zugehörige Fibrinogenrestkonzentration, so ergibt sich für äquipotente Dosen bei K2P ein Restgehalt an Fibrinogen von 92,5 % gegenüber 38,6 % bei Actilyse[R]. Auch für Plasminogen und $\alpha_2$-Antiplasmin findet sich eine dosisabhängige Erniedrigung der Restgehalte 2 h nach Injektion, die bei Actilyse[R] ausgeprägter ist als bei K2P. Lediglich die Plättchenkonzentration ist bei beiden Substanzen nahezu unbeeinflußt.

### 4. Einfluß auf die Blutungszeit am Hund

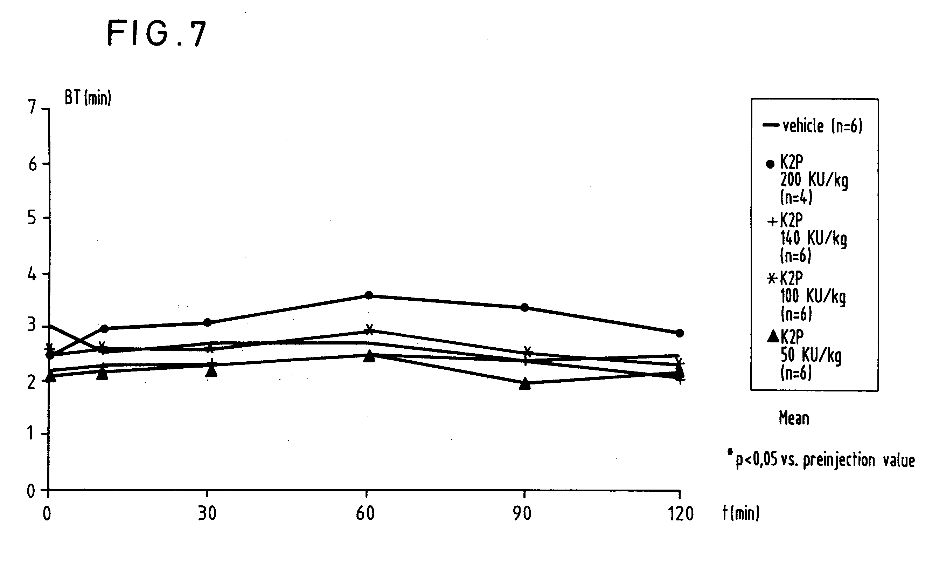

Die intravenöse Injektion von K2P ergab keine statistisch signifikante Verlängerung der Blutungszeit im Vergleich mit dem Kontrollwert vor Injektion in allen 4 untersuchten Dosen. Dagegen verlängerte Actilyse[R] in Dosen von 1130 und 1600 kU/kg KG die Blutungszeit statistisch signifikant (Fig. 6 und 7).

### 5. Gesamtbeurteilung

An dem beschriebenen Hundemodell der Koronararterienthrombose erwies sich K2P als ein Thrombolytikum, das nach intravenöser Bolusinjektion eine 100 %ige Reperfusionsrate erzielen kann, ohne die Fibrinogenkonzentration stark zu beeinflussen und ohne die Blutungszeit signifikant zu verlängern. Im Vergleich mit Actilyse[R] als Stand der Technik zeigte sich K2P in der thrombolytischen Potenz nach intravenöser Bolusinjektion deutlich überlegen (Faktor 11,5). Die Untersuchung des pharmakokinetischen Profils von K2P ergab weiterhin, daß im Vergleich mit Actilyse[R] die Clearance von K2P als Ausdruck für die langsamere Entfernung aus dem Plasma neunfach verlangsamt ist.

**Tab. 3**       Thrombolyseparameter an einem Hundemodell der Koronararterienthrombose nach i.v. Bolus-injektion (über 1 min) von Placebo oder steigender Dosen von Actilyse[R] oder K2P

| Substanz | Dosis (IU/kg KG) | n | Reperfusionsrate | Reperfusionszeit (min) | Feuchtgewicht des Restthrombus (mg) | Reokklusionsrate |
|---|---|---|---|---|---|---|
| Placebo | – | 6 | 0/6 | / | $9.6 \pm 1.2$ | / |
| Actilyse[R] | 1 600 000 | 6 | 6/6 | $28 \pm 11$ | $2,2 \pm 0,7$ | 4/6 |
| | 1 130 000 | 6 | 4/6 | $14 \pm 3$ | $4,4 \pm 0,5$ | 4/4 |
| | 800 000 | 6 | 2/6 | $35 \pm 38$ | $5,5 \pm 0,4$ | 1/2 |
| | 200 000 | 6 | 0/6 | / | $8,8 \pm 1,3$ | / |
| K2P | 200 000 | 6 | 6/6 | $29 \pm 6$ | $2,5 \pm 0,3$ | 6/6 |
| | 140 000 | 6 | 4/6 | $15 \pm 6$ | $4,4 \pm 1,4$ | 4/4 |
| | 100 000 | 6 | 3/6 | $30 \pm 14$ | $7,4 \pm 1,1$ | 2/3 |
| | 50 000 | 6 | 2/6 | $31 \pm 4$ | $5,5 \pm 0,8$ | 2/2 |

Mittelwert $\pm$ SEM

EP 0 382 174 B1

Tab. 4    Pharmakokinetische Parameter, abgeleitet aus Computerberechnungen von Plasmakonzentrations-Zeit-Daten auf Basis der t-PA-Aktivität, an narkotisierten Hunden nach i.v. Bolusinjektion von 200 000 IU/kg KG Actilyse$^R$ oder K2P

| Substanz | $t_{1/2\alpha}$ (min) | $t_{1/2\beta}$ (min) | $C_{inj.}$ (IU/ml) | $V_c$ (ml/kg) | $Cl_{tot}$ (ml·kg$^{-1}$·min$^{-1}$) | $AUC_{extrapol.}$ (IU·ml$^{-1}$·h) |
|---|---|---|---|---|---|---|
| Actilyse$^R$ (n = 6) | 1,6 0,2 | 14,6 6,6 | 1674 ±504 | 106,1 ±37 | 41,6 ±11,4 | 84,1 ±23,8 |
| K2P (n = 6) | 7,2 ±1,1 (n=4) | 15,0 ±4,3 | 3177 ±817 | 64,7 ±19,7 | 4,6 ±1,3 | 803 250 |

Mittelwert ± Standardabweichung

$V_c$                =    Verteilungsvolumen des zentralen Kompartiments
$Cl_{tot}$           =    Gesamtplasmaclearance
$AUC_{extrapol.}$    =    "area under the curve"; extrapolierte Fläche unter der Plasmakonzentrations-Zeit-Kurve

**Tab. 5**     Gerinnungsparameter und Thrombozytenzahl 2 h nach i.v. Bolusinjektion (über 1 min) von Placebo oder steigender Dosen von Actilyse[R] oder K2P an einem Hundemodell der Koronararterienthrombose

| Substanz | Dosis (IU/kg KG) | n | Fibrinogen (%) | Plasminogen (%) | $\alpha_2$-Antiplasmin (%) | Plättchenzahl (%) |
|---|---|---|---|---|---|---|
| Placebo | – | 6 | 106 ± 2 | 103 (n=4) | 92 ± 11 (n=4) | 101 ± 3 |
| Actilyse[R] | 1 600 000 | 6 | 3 ± 0 | 46 ± 2 | 19 ± 11 | 119 ± 10 |
| | 1 130 000 | 6 | 16 ± 13 | 59 ± 6 | 45 ± 6 | 110 ± 10 |
| | 800 000 | 6 | 86 ± 4 | 87 ± 3 | 64 ± 5 | 104 ± 6 |
| | 200 000 | 6 | 97 ± 3 | 90 ± 3 | 86 ± 4 | 109 ± 4 |
| K2P | 200 000 | 6 | 81 ± 10 | 74 ± 8 | 40 ± 6 | 106 ± 8 |
| | 140 000 | 6 | 93 ± 1 | 81 ± 1 | 50 ± 7 | 104 ± 5 |
| | 100 000 | 6 | 92 ± 3 | 88 ± 3 | 77 ± 7 | 101 ± 3 |
| | 50 000 | 6 | 95 ± 3 | 90 ± 2 | 84 ± 12 | 118 ± 11 |

Mittelwert ± SEM

Gerinnungswerte und Thrombozytenzahl als % des Ausgangswertes vor Injektion

EP 0 382 174 B1

**Patentansprüche**
**Patentansprüche für folgende Vertragsstaaten : AT, BE, CH, LI, DE, DK, FR, GB, IT, LU, NL, SE**

1. Gewebs-Plasminogenaktivator (t-PA)-Derivat,
   **dadurch gekennzeichnet,**
   daß es nicht glykosyliert ist und aus der folgenden Aminosäuresequenz besteht:

```
     (M)
   1   SYQGNSDCYF GNGSAYRGTH SLTESGASCL PWNSMILIGK VYTAQNPSAQ
  51   ALGLGKHNYC RNPDGDAKPW CHVLKNRRLT WEYCDVPSCS TCGLRQYSQP
 101   QFRIKGGLFA DIASHPWQAA IFAKHRRSPG ERFLCGGILI SSCWILSAAH
 151   CFQERFPPHH LTVILGRTYR VVPGEEEQKF EVEKYIVHKE FDDDTYDNDI
 201   ALLQLKSDSS RCAQESSVVR TVCLPPADLQ LPDWTECELS GYGKHEALSP
 251   FYSERLKEAH VRLYPSSRCT SQHLLNRTVT DNMLCAGDTR SGGPQANLHD
 301   ACQGDSGGPL VCLNDGRMTL VGIISWGLGC GQKDVPGVYT KVTNYLDWIR
 351   DNMRP
```

die am Aminoende noch durch M verlängert sein kann.

2. DNA-Sequenz, **dadurch gekennzeichnet,** daß sie für ein t-PA-Derivat nach Anspruch 1 kodiert und die folgende Sequenz enthält:

```
   1   ATGTCTTACCAAGGAAACAGTGACTGCTACTTTGGGAATGGGTCAGCCTACCGTGGCACG.   60
  61   CACAGCCTCACCGAGTCGGGTGCCTCCTGCCTCCCGTGGAATTCCATGATCCTGATAGGC   120
 121   AAGGTTTACACAGCACAGAACCCCAGTGCCCAGGCACTGGGCCTGGGCAAACATAATTAC   180
 181   TGCCGGAATCCTGATGGGGATGCCAAGCCCTGGTGCCACGTGCTGAAGAACCGCAGGCTG   240
 241   ACGTGGGAGTACTGTGATGTGCCCTCCTGCTCCACCTGCGGCCTGAGACAGTACAGCCAG   300
 301   CCTCAGTTTCGCATCAAAGGAGGGCTCTTCGCCGACATCGCCTCCCACGCCTGGCAGGCT   360
 361   GCCATCTTTGCCAAGCACAGGAGGTCGCCCGGAGAGCGGTTCCTGTGCGGGGGCATACTC   420
 421   ATCAGCTCCTGCTGGATTCTCTCTGCCGCCCACTGCTTCCAGGAGAGGTTTCCGCCCCAC   480
 481   CACCTGACGGTGATCTTGGGCAGAACATACCGGGTGGTCCCTGGCGAGGAGGAGCAGAAA   540
 541   TTTGAAGTCGAAAAATACATTGTCCATAAGGAATTCGATGATGACACTTACGACAATGAC   600
 601   ATTGCGCTGCTGCAGCTGAAATCGGATTCGTCCCGCTGTGCCCAGGAGAGCAGCGTGGTC   660
```

```
661   CGCACTGTGTGCCTTCCCCCGGCGGACCTGCAGCTGCCGGACTGGACGGAGTGTGAGCTC   720

721   TCCGGCTACGGCAAGCATGAGGCCTTGTCTCCTTTCTATTCGGAGCGGCTGAAGGAGGCT   780

781   CATGTCAGACTGTACCCATCCAGCCGCTGCACATCACAACATTTACTTAACAGAACAGTC   840

841   ACCGACAACATGCTGTGTGCTGGAGACACTCGGAGCGGCGGGCCCCAGGCAAACTTGCAC   900

901   GACGCCTGCCAGGGCGATTCGGGAGGCCCCCTGGTGTGTCTGAACGATGGCCGCATGACT   960

961   TTGGTGGGCATCATCAGCTGGGGCCTGGGCTGTGGACAGAAGGATGTCCCGGGTGTGTAC   1020

1021  ACAAAGGTTACCAACTACCTAGACTGGATTCGTGACAACATGCGACCG   1068
```

3. Expressionsplasmid, **dadurch gekennzeichnet**, daß es die DNA-Sequenz nach Anspruch 2, oder eine im Rahmen der Degeneration des genetischen Codes davon verschiedene DNA-Sequenz, die für ein Protein gemäß Anspruch 1 kodiert, enthält.

4. Plasmid pA27.3, enthaltend eine DNA-Sequenz gemäß Anspruch 2 und schematisch dargestellt in Fig. 1.

5. Plasmid pA 27 fd, enthaltend eine DNA-Sequenz gemäß Anspruch 2, einligiert in einen Vektor, der in Wirtszellen in hoher Kopienzahl vorliegt und hergestellt wie in Beispiel 5 beschrieben.

6. Verfahren zur Herstellung eines Plasmids nach einem der Ansprüche 3 bis 5, **dadurch gekennzeichnet,** daß man eine DNA-Sequenz, die für das gesamte t-PA-Protein oder für ein Derivat davon, welches über die Kringel II-und die Protease-Domänen hinaus noch weitere Bereiche des t-PA-Proteins aufweist, kodiert, in ein Plasmid einbringt und über gerichtete Mutagenese diejenigen Bereiche entfernt, die für Aminosäuren kodieren, die in dem t-PA-Derivat nach Anspruch 1 nicht vorhanden sind.

7. Verfahren nach Anspruch 6, **dadurch gekennzeichnet,** daß man als DNA-Sequenz für das t-PA-Protein oder ein Derivat davon die entsprechende cDNA verwendet.

8. Verfahren nach Anspruch 6 oder 7,
**dadurch gekennzeichnet,**
daß man das Plasmid pA 27.3 gemäß Anspruch 4, oder das Plasmid pA 27 fd gemäß Anspruch 5, verwendet.

9. Verfahren zur Herstellung eines t-PA-Derivats nach Anspruch 1,
**dadurch gekennzeichnet,**
daß man ein Plasmid nach einem der Ansprüche 3 bis 5 in geeignete Wirtszellen transformiert und das Expressionsprodukt aus dem Kulturmedium oder nach Aufschluß der Wirtszellen gewinnt.

10. Verfahren nach Anspruch 9,
**dadurch gekennzeichnet,**
daß man als Wirtszellen Prokaryontenzellen, insbesondere E.coli, verwendet.

11. Verfahren nach Anspruch 10,
**dadurch gekennzeichnet,**
daß man zur Erhöhung der Ausbeute an aktivem Protein gebildete "inclusion bodies" abtrennt, diese durch Behandlung mit Guanidinhydrochlorid solubilisiert, anschließend mit oxidiertem Glutathion derivatisiert und zuletzt das t-PA-Derivat durch Zugabe von L-Arginin und GSH renaturiert.

12. Verfahren nach Anspruch 11, **dadurch gekennzeichnet,** daß man nach der Renaturierung K2P im Renaturierungsansatz konzentriert und anschließend eine chromatographische Reinigung mittels Affinitätschromatographie durchführt.

EP 0 382 174 B1

**13.** Verfahren nach Anspruch 12, **dadurch gekennzeichnet,** daß man in Gegenwart von 10 bis 1000 mmol/l L-Arginin arbeitet.

**14.** Verfahren nach Anspruch 13, **dadurch gekennzeichnet,** daß man die chromatographische Reinigung mit dem Konzentrat des Renaturierungsansatzes, das 10 bis 1000 mmol/l, bevorzugt 600 bis 800 mmol/l L-Arginin enthält, über eine ETI-Adsorbersäule durchführt.

**15.** Verfahren nach Anspruch 14, **dadurch gekennzeichnet,** daß man das zur chromatographischen Reinigung eingesetzte Konzentrat auf einen pH-Wert von 7,5 bis 8,6 einstellt.

**16.** Verfahren nach Anspruch 14 oder 15, **dadurch gekennzeichnet,** daß man die Elution bei einem pH-Wert von 3 bis 5,5 durchführt.

**17.** Arzneimittel zur fibrinolytischen Behandlung, enthaltend ein t-PA-Derivat nach Anspruch 1.

**Patentansprüche für folgende Vertragsstaaten : ES, GR**

**1.** Verfahren zur Herstellung eines Gewebs-Plasminogenaktivator (t-PA)-Derivats, das nicht glykosyliert ist und aus der folgenden Aminosäuresequenz besteht:

```
      (M)
    1   SYQGNSDCYF  GNGSAYRGTH  SLTESGASCL  PWNSHILIGK  VYTAQNPSAQ
   51   ALGLGKHNYC  RNPDGDAKPW  CHVLKNRRLT  WEYCDVPSCS  TCGLRQYSQP
  101   QFRIKGGLFA  DIASHPWQAA  IFAKHRRSPG  ERFLCGGILI  SSCWILSAAH
  151   CFQERFPPHH  LTVILGRTYR  VVPGEEEQKF  EVEKYIVHKE  FDDDTYDNDI
  201   ALLQLKSDSS  RCAQESSVVR  TVCLPPADLQ  LPDWTECELS  GYGKHEALSP
  251   FYSERLKEAH  VRLYPSSRCT  SQHLLNRTVT  DNMLCAGDTR  SGGPQANLHD
  301   ACQGDSGGPL  VCLNDGRMTL  VGIISWGLGC  GQKDVPGVYT  KVTNYLDWIR
  351   DNMRP
```

die am Aminoende noch durch M verlängert sein kann,
**dadurch gekennzeichnet,**
daß man ein Expressionsplasmid, das die DNA-Sequenz

19

```
   1   ATGTCTTACCAAGGAAACAGTGACTGCTACTTTGGGAATGGGTCAGCCTACCGTGGCACG .  60

  61   CACAGCCTCACCGAGTCGGGTGCCTCCTGCCTCCCGTGGAATTCCATGATCCTCATAGGC   120

 121   AAGGTTTACACAGCCACAGAACCCCAGTGCCCAGGCACTGGGCCTGGGCAAACATAATTAC   180

 181   TGCCGGAATCCTGATGGGGATCCCAAGCCCTGGTGCCACGTGCTGAAGAACCGCAGGCTG   240

 241   ACGTGGGAGTACTGTGATGTGCCCTCCTGCTCCACCTGCGGCCTGAGACAGTACAGCCAG   300

 301   CCTCAGTTTCGCATCAAAGGAGGGCTCTTCGCCGACATCGCCTCCCACCCCTGGCAGGCT   360

 361   GCCATCTTTGCCAAGCACAGGAGGTCGCCCCGAGAGCGGTTCCTGTGCGGCGGCATACTC   420

 421   ATCAGCTCCTGCTGGATTCTCTCTGCCGCCCACTGCTTCCAGGAGAGGTTTCCGCCCCAC   480

 481   CACCTCACCGTGATCTTGGGCAGAACATACCGGGTGGTCCCTGGCCAGGAGGAGCAGAAA   540

 541   TTTGAAGTCGAAAAATACATTGTCCATAAGGAATTCGATGATGACACTTACGACAATGAC   600

 601   ATTGCGCTGCTGCAGCTGAAATCGGATTCGTCCCGCTGTGCCCAGGAGAGCAGCGTCGTC   660
```

```
 661   CGCACTGTGTGCCTTCCCCCGGCGGACCTGCAGCTGCCGGACTGGACGGAGTGTGAGCTC   720

 721   TCCCGCTACGGCAAGCATGAGGCCTTGTCTCCTTTCTATTCGGAGCGGCTGAAGGAGGCT   780

 781   CATGTCAGACTGTACCCATCCAGCCGCTGCACATCACAACATTTACTTAACAGAACAGTC   840

 841   ACCGACAACATGCTGTGTGCTGGAGACACTCGGCAGCGGCGGGCCCCAGGCAAACTTGCAC   900

 901   GACGCCTGCCAGGGCGATTCGGGAGGCCCCCTGGTGTGTGTCTGAACGATGGCCGCATGACT   960

 961   TTGGTGGGCATCATCAGCTGGGGCCTGGGCTGTGGACAGAAGGATGTCCCCGGGTGTGTAC  1020

1021   ACAAAGGTTACCAACTACCTAGACTGGATTCGTGACAACATGCGACCG  1068
```

oder eine im Rahmen der Degeneration des genetischen Codes davon verschiedene DNA-Sequenz, die für ein entsprechendes t-PA-Derivat kodiert, enthält, in geeignete Wirtszellen einbringt und das Expressionsprodukt aus dem Kulturmedium oder nach Aufschluß der Wirtszellen gewinnt.

2.  Verfahren nach Anspruch 1,
    **dadurch gekennzeichnet,**
    daß man das Plasmid pA27.3, schematisch dargestellt in Figur 1, verwendet.

3.  Verfahren nach Anspruch 1,
    **dadurch gekennzeichnet,**
    daß man das Plasmid pA27fd, hergestellt wie in Beispiel 5 beschrieben, verwendet.

4.  Verfahren nach Anspruch 1, 2 oder 3,
    **dadurch gekennzeichnet,**
    daß man als Wirtszellen Prokaryontenzellen, insbesondere E.coli verwendet.

5.  Verfahren nach Anspruch 4,
    **dadurch gekennzeichnet,**
    daß man zur Erhöhung der Ausbeute an aktivem Protein gebildete "inclusion bodies" abtrennt, diese durch Behandlung mit Guanidinhydrochlorid solubilisiert, anschließend mit oxidiertem Glutathion derivatisiert und zuletzt das t-PA-Derivat durch Zugabe von L-Arginin und GSH renaturiert.

EP 0 382 174 B1

**6.** Verfahren nach Anspruch 5,
**dadurch gekennzeichnet,**
daß man nach der Renaturierung das t-PA-Derivat im Renaturierungsansatz konzentriert und anschließend eine chromatographische Reinigung mittels Affinitätschromatographie durchführt.

**7.** Verfahren nach Anspruch 6,
**dadurch gekennzeichnet,**
daß man in Gegenwart von 10 bis 1000 mmol/l L-Arginin arbeitet.

**8.** Verfahren nach Anspruch 7,
**dadurch gekennzeichnet,**
daß man die chromatographische Reinigung mit dem Konzentrat des Renaturierungsansatzes, das 10 bis 1000 mmol/l, bevorzugt 600 bis 800 mmol/l L-Arginin enthält, über eine ETI-Adsorbersäule durchführt.

**9.** Verfahren nach Anspruch 8,
**dadurch gekennzeichnet,**
daß man das zur chromatographischen Reinigung eingesetzte Konzentrat auf einen pH-Wert von 7,5 bis 8,6 einstellt.

**10.** Verfahren nach Anspruch 8 oder 9,
**dadurch gekennzeichnet,**
daß man die Elution bei einem pH-Wert von 3 bis 5,5 durchführt.

**11.** Verfahren zur Herstellung eines Expressionsplasmids, das die DNA-Sequenz

```
   1  ATGTCTTACCAAGGAAACACTGACTGCTACTTTGGGAATGGGTCAGCCTACCGTCGCACG   60
  61  CACAGCCTCACCGAGTCGCGTGCCTCCTGCCTCCCGTGGAATTCCATGATCCTGATAGGC  120
 121  AAGGTTTACACAGCCACAGAACCCCAGTGCCCAGGCACTGGGCCTCGGCAAACATAATTAC  180
 181  TGCCCGAATCCTGATCGGGCATGCCAAGCCCTGGTGCCACGTGCTGAAGAACCGCAGGCTG  240
 241  ACGTGGGAGTACTGTGATGTGCCCTCCTGCTCCACCTGCGGGCCTGACACAGTACAGCCAG  300
 301  CCTCAGTTTCGCATCAAAGGAGGGCTCTTCGCCCGACATCCCCTCCCACCCCTGCCAGGCT  360
 361  GGCATCTTTGCCAAGCACAGCAGGTCGCCCCGGAGAGCGGTTCCTGTGCCGGGGGCATACTC  420
 421  ATCAGCTCCTGCTGGATTCTCTCTGCCGCCCACTGCTTCCAGGAGACGTTTCCGCCCCAC  480
 481  CACCTGACGGTGATCTTGGGCAGAACATACCGGGTGGTCCCTGGCGAGGAGGAGCACAAA  540
 541  TTTGAAGTCGAAAAATACATTGTCCATAAGGAATTCGATGATGACACTTACGACAATGAC  600
 601  ATTGCCCTGCTCCAGCTGAAATCGGATTCGTCCCCGCTGTGCCCAGGAGAGCAGCGTCGTC  660
 661  CGCACTGTGTGCCTTCCCCCGCCGGCACCTGCAGCTGCCGGACTGGACGGAGTGTCAGCTC  720
 721  TCCGGCTACGGCAAGCATGAGGCCTTGTCTCCTTTCTATTCGGAGCGGCTCAAGGAGGCT  780
 781  GATGTCAGACTCTACCCATCCAGCCGCTGCACATCACAACATTTACTTAACAGAACAGTC  840
 841  ACCCACAACATGCTGTGTGCTGGAGACACTCGGAGCGGCGGGCCCCAGGCAAACTTGCAC  900
 901  GACGCCTGCCAGGGCGATTCGGGAGGCCCCCTGGTGTGTCTCAACGATGGCCGCATGACT  960
 961  TTGGTGCGGCATCATCAGCTGGGGCCCTGGGCTGTGGACAGAAGCATGTCCCCGGGTGTCTAC  1020
1021  ACAAAGGTTACCAACTACCTAGACTGGATTCGTGACAACATGCGACCG  1068
```

oder eine im Rahmen der Degeneration des genetischen Codes davon verschiedene DNA-Sequenz enthält, die für ein t-PA-Derivat kodiert, das nicht glykosyliert ist und die folgende Aminosäuresequenz aufweist

```
     (M)
   1   SYQGNSDCYF GNGSAYRGTH SLTESGASCL PWNSHILIGK VYTAQNPSAQ
  51   ALGLGKHNYC RNPDGDAKPW CHVLKNRRLT WEYCDVPSCS TCGLRQYSQP
 101   QFRIKGGLFA DIASHPWQAA IFAKHRRSPG ERFLCGGILI SSCWILSAAH
 151   CFQERFPPHH LTVILGRTYR VVPGEEEQKF EVEKYIVHKE FDDDTYDNDI
 201   ALLQLKSDSS RCAQESSVVR TVCLPPADLQ LPDWTECELS GYGKHEALSP
 251   FYSERLKEAH VRLYPSSRCT SQHLLNRTVT DNMLCAGDTR SGGPQANLHD
 301   ACQGDSGGPL VCLNDGRHTL VGIISWGLGC GQKDVPGVYT KVTNYLDWIR
 351   DNMRP
```

oder zur Herstellung von Plasmid pA27.3 gemäß Figur 1 oder pA27fd, hergestellt nach Beispiel 5,
**dadurch gekennzeichnet,**
daß man eine DNA-Sequenz, die für das gesamte t-PA-Protein oder für ein Derivat davon, welches über die Kringel II- und die Protease-Domänen hinaus noch weitere Bereiche des t-PA-Proteins aufweist, kodiert, in ein Plasmid einbringt und über gerichtete Mutagenese diejenigen Bereiche entfernt, die für Aminosäuren kodieren, die in dem t-PA-Derivat nicht vorhanden sind.

12. Verfahren nach Anspruch 11,
**dadurch gekennzeichnet,**
daß man als DNA-Sequenz für das t-PA-Protein oder ein Derivat davon die entsprechende cDNA verwendet.

**Claims**

**Claims for the following Contracting States : AT, BE, CH, LI, DE, DK, FR, GB, IT, LU, NL, SE**

1. Derivative of tissue-type plasminogen activator (t-PA), wherein the derivative is not glycosylated and consists of the following amino acid sequence:

```
       (M)
   1   SYQGNSDCYF GNGSAYRGTH SLTESGASCL PWNSHILIGK VYTAQNPSAQ
  51   ALGLGKHNYC RNPDGDAKPW CHVLKNRRLT WEYCDVPSCS TCGLRQYSQP
 101   QFRIKGGLFA DIASHPWQAA IFAKHRRSPG ERFLCGGILI SSCWILSAAH
 151   CFQERFPPHH LTVILGRTYR VVPGEEEQKF EVEKYIVHKE FDDDTYDNDI
 201   ALLQLKSDSS RCAQESSVVR TVCLPPADLQ LPDWTECELS GYGKHEALSP
 251   FYSERLKEAH VRLYPSSRCT SQHLLNRTVT DNMLCAGDTR SGGPQANLHD
 301   ACQGDSGGPL VCLNDGRHTL VGIISWGLGC GQKDVPGVYT KVTNYLDWIR
 351   DNMRP
```

which can be extended by M at the amino terminal end.

2. DNA sequence, **wherein**
   it codes for a t-PA derivative as claimed in claim 1 and contains the following sequence:

```
   1  ATGTCTTACCAAGCAAACAGTCACTCCTACTTTCCCAATCGGTCAGCCTACCGTCGCCACG   60
  61  CACAGCCTCACCCAGTCGCGTGCCTCCTCCCTCCCGTCGAATTCCATGATCCTGATAGGC  120
 121  AAGGTTTACACACCACAGAACCCCAGTGCCCAGGCACTCGGCCTCGGCAAACATAATTAC  180
 181  TCCCCGAATCCTGATGGGGATGCCAAGCCCTGGTGCCACGTGCTGAAGAACCGCAGGCTG  240
 241  ACCTGGGAGTACTGTGATGTGCCCTCCTGCTCCACCTGCGGCCTGAGACAGTACAGCCAG  300
 301  CCTCAGTTTCCCATCAAAGGAGGGCTCTTCGCCCGACATCGCCTCCCACCCCTCGCAGGCT  360
 361  GCCATCTTTGCCAAGCACAGGAGGTCGCCCCGGAGAGCCGGTTCCTGTGCGGGGGCATACTC  420
 421  ATCAGCTCCTGCTGGATTCTCTCTGCCGCCCACTGCTTCCAGGAGAGGTTTCCGCCCCAC  480
 481  CACCTCACGGTCATCTTGGGCACAACATACCGGGTGGTCCCTGGCGAGGAGGAGCAGAAA  540

 541  TTTGAAGTCGAAAAATACATTGTCCATAAGGAATTCGATCATGACACTTACCACAATGAC   600
 601  ATTGCGCTGCTGCAGCTGAAATCGGATTCGTCCCGCTGTGCCCAGGAGAGCAGCGTCCTC   660
 661  CGCACTGTGTGCCTTCCCCCGGCCGACCTGCAGCTGCCCGACTGGACGGACTGTCAGCTC   720
 721  TCCGGCTACGGCAAGCATGAGGCCTTGTCTCCTTTCTATTCGGAGCGGCTCAAGGAGGCT   780
 781  CATGTCAGACTCTACCCATCCAGCCGCTGCACATCACAACATTTACTTAACAGAACAGTC   840
 841  ACCCACAACATGCTGTCTGCTGGAGACACTCCGAGCGGCGGGCCCCAGGCAAACTTGCAC   900
 901  GACGCCTGCCAGGGCGATTCCGGAGGCCCCCTGCTGTGTCTGAACGATGGCCGCATGACT   960
 961  TTGGTGGGCATCATCAGCTGGGGCCTGGGCTGTGGACAGAAGGATGTCCCCGGTGTCTAC   1020
1021  ACAAAGGTTACCAACTACCTAGACTGGATTCGTGACAACATGCGACCG  1068
```

3. Expression plasmid, **wherein**
   it contains the DNA sequence as claimed in claim 2 or a DNA sequence which differs within the scope of the degeneracy of the genetic code which codes for a protein as claimed in claim 1.

4. Plasmid pA27.3 which contains a DNA sequence as claimed in claim 2 and is shown schematically in Fig. 1.

5. Plasmid pA27 fd containing a DNA sequence as claimed in claim 2 which is ligated in a vector which is present in host cells in a high copy number and is prepared as described in example 5.

6. Process for the construction of a plasmid as claimed in one of the claims 3 to 5,
   **wherein** a DNA sequence which codes for the whole t-PA protein or a derivative thereof containing further regions of the t-PA protein in addition to the kringle II and the protease domains is incorporated into a plasmid and those domains which code for amino acids which are not present in the t-PA derivative as claimed in claim 1 are deleted by site-directed mutagenesis.

7. Process as claimed in claim 6, **wherein** the corresponding cDNA is used as the DNA sequence for the t-PA protein or a derivative thereof.

8. Process as claimed in claims 6 or 7,
   **wherein** plasmid pA27.3 as claimed in claim 4 or plasmid pA27 fd as claimed in claim 5 are used.

EP 0 382 174 B1

9. Process for the production of a t-PA derivative as claimed in claim 1, **wherein** a plasmid according to one of the claims 3 to 5 is transformed into suitable host cells and the expression product is isolated from the culture medium or after lysis of the host cells.

10. Process as claimed in claim 9, **wherein** prokaryotic cells and in particular E. coli are used as host cells.

11. Process as claimed in claim 10, **wherein** the yield of active protein is increased by isolating the "inclusion bodies" that form and solubilizing them by treatment with guanidine hydrochloride, followed by derivatization with oxidized glutathione and finally renaturation the t-PA derivative by addition of L-arginine and GSH.

12. Process as claimed in claim 11, **wherein** after the renaturation K2P is concentrated in the renaturation preparation and subsequently a chromatographic purification is carried out by means of affinity chromatography.

13. Process as claimed in claim 12, **wherein** one works in the presence of 10 to 1000 mmol/l L-arginine.

14. Process as claimed in claim 13, **wherein** the chromatographic purification with the concentrate of the renaturation preparation, which contains 10 to 1000 mmol/l, preferably 600 to 800 mmol/l L-arginine, is carried out over an ETI adsorber column.

15. Process as claimed in claim 14, **wherein** the concentrate used for the chromatographic purification is adjusted to a pH value of 7.5 to 8.6.

16. Process as claimed in claim 14 or 15,
    **wherein** the elution is carried out at a pH value of 3 to 5.5.

17. Pharmaceutical agent for fibrinolytic treatment containing a t-PA derivative as claimed in claim 1.

**Claims for the following Contracting States : ES, GR**

1. Process for the production of a derivative of tissue-type plasminogen activator (t-PA) which is not glycosylated and consists of the following amino acid sequence:

```
(M)
  1  SYQGNSDCYF GNGSAYRGTH SLTESGASCL PWNSMILICK VYTAQNPSAQ

 51  ALGLGKHNYC RNPDGDAKPW CHVLKNRRLT WEYCDVPSCS TCGLRQYSQP

101  QFRIKGGLFA DIASHPWQAA IFAKHRRSPG ERFLCGGILI SSCWILSAAH

151  CFQERFPPHH LTVILGRTYR VVPGEEEQKF EVEKYIVHKE FDDDTYDNDI

201  ALLQLKSDSS RCAQESSVVR TVCLPPADLQ LPDWTECELS GYGKHEALSP

251  FYSERLKEAH VRLYPSSRCT SQHLLNRTVT DNMLCAGDTR SGGPQANLHD

301  ACQGDSGGPL VCLNDGRMTL VGIISWGLGC GQKDVPGVYT KVTNYLDWIR

351  DNMRP
```

which can be extended by M at the amino terminal end,
**wherein**
an expression plasmid which contains the DNA sequence

24

```
   1  ATGTCTTACCAACGAAACAGTGACTGCTACTTTGCGAATGGGTCAGCCTACCGTGCCACG .  60
  61  CACACCCTCACCGAGTCGGGTGCCTCCTCCCTCCCGTGCAATTCCATGATCGTGATAGGC  120
 121  AAGCTTTACACAGGCACAGAACCCCAGTGCCCACGCACTGGGCCTCGGGCAAACATAATTAC  180
 181  TGCCCGAATGGTCATGGGCGATGGGAAGGGCTGGTGGCCACGTGGTGAAGAACCGGCAGGGCTG  240
 241  ACGTGGGACTACTGTGATGTGGGGCTGGTGGTGGAACCTGGGGGGCTGAGACAGTACAGGGCAG  300
 301  GGTGAGTTTGGGATCAAAGGAGGGGGCTGTTGGGGGGACATGGGGCTGGGACCGGCTGGGCAGGGCT  360
 361  GGGATGTTTGGGAAGGGACAGGGAGGTGGGGGGGGGCAGGAGGGGGGTTGGTGTGGGGGGGGGGCATAGTG  420
```

```
 421  ATGAGGCTGGTGGTGGGATTGTGTGTGGGGGGGGGGCACTGGTTGGCACGAGAGGGGTTTGGGGGGGGCAC  480
 481  CACGTGAGGGGTGATGTTGGGGGCACAAGATAGGGGGGTGGTGGGGGTGGCGAGGGAGGGAGGAGCAGAAA  540
 541  TTTGAAGTGGAAAAATAGATTGTGGCATAAGGAATTGGCATGATGAGACACTTAGGACAATGAG .  600
 601  ATTGGGGGTGGGTGGCAGGCTGAAATGGGATTGGTGTGGGGGCTGTGGCCCACGAGAGGGAGGGGCGTGGGTG  660
 661  GGGGCACTGTGTGGGCGTTGGGGGGGGGGGGGGGGACGTGGCAGGGTGGGGGGGGACTGGCAGGGGGGGGTGTGAGGGGTG  720
 721  TGGGGGGCTAGGGGCAAGGGCATGAGGGGGCTTTGTGTGTGGGGTTTGGTATTGGGGGAGGGGGCGGGGGGTGAAGGGCAGGGGGTGGGCT  780
 781  CATGTGGGCAGACTGGTGACGGCATGGCAGGGGGGGGGGGGGCTGGGGGGACATGGCACAAGGCATTTACTTAAGGAGAAGCAGTG  840
 841  AGGGACAAGGCATGGGGGGGGGGGGGGGGGGGGGGGGGGACAGACATGGGGGGGGGGGGGGGGGGGGGGGGGGCCCCCAGGGCCAAAGGCTTTGGGCAC .  900
 901  GACGGGGCTGGGCACGGGGGGCGATTGGGGGGGCAGGGCCCCGGGGGGGCTGGGGGGCTGTGTGTGGTGTGTGGTGTGGGCAACCATGGGGGCCCCCATGACT  960
 961  TTGGGGTGGGGGGGGCATCATGGAGGGGCTGGGGGGGGGGGCTGGGGGGGGCTGTGGGGACACAAGGGCATGGGTGCCCCGGGGCTGTGTAC  1020
1021  ACAAAGGGTTACCAAGGCTACGGGTACGGGGGTAGACTGGGATTGGGGGTGGGCAAGGGACATGGGGGCACGGGG  1068
```

or a DNA sequence which differs within the scope of the degeneracy of the genetic code which codes for a respective t-PA derivative, is incorporated into suitable host cells and the expression product is isolated from the culture medium or after lysing the host cells.

2.  Process as claimed in claim 1,
    **wherein**
    plasmid pA27.3 which is shown schematically in Figure 1 is used.

3.  Process as claimed in claim 1,
    **wherein**
    plasmid pA27fd, prepared as described in example 5, is used.

4.  Process as claimed in claim 1, 2 or 3,
    **wherein**
    prokaryotic cells, in particular E. coli, are used as the host cells.

5.  Process as claimed in claim 4,
    **wherein**
    the yield of active protein is increased by isolating the "inclusion bodies" that form and solubilizing them by treatment with guanidine hydrochloride, followed by derivatization with oxidized glutathione and finally renaturation of the t-PA derivative by addition of L-arginine and GSH.

6. Process as claimed in claim 5,
**wherein**
after the renaturation the t-PA derivative is concentrated in the renaturation preparation and subsequently a chromatographic purification is carried out by means of affinity chromatography.

7. Process as claimed in claim 6,
**wherein**
one works in the presence of 10 to 1000 mmol/l L-arginine.

8. Process as claimed in claim 7,
**wherein**
the chromatographic purification with the concentrate of the renaturation preparation, which contains 10 to 1000 mmol/l, preferably 600 to 800 mmol/l L-arginine, is carried out over an ETI adsorber column.

9. Process as claimed in claim 8,
**wherein**
the concentrate used for the chromatographic purification is adjusted to a pH value of 7.5 to 8.6.

10. Process as claimed in claim 8 or 9,
**wherein**
the elution is carried out at a pH value of 3 to 5.5.

11. Process for the construction of an expression plasmid which contains the DNA sequence

```
  1  ATGTCTTACCAAGCAAACACTCACTCCTACTTTGCGAATGGGTCAGCCTACCGTCGCACCG   60
 61  CACACCCTCACCCACTCCCGTGCCTCCTGCCTCCCGTGGAATTCCATGATCCTCATAGGCC  120
121  AAGCTTTACACACCACAGAACCCCACTGCCCAGGCACTGGGCCTCGGCAAACATAATTAC   180
181  TGCCCGAATCCTGATCGGGATGCCAAGCCCTGCTGCCACGTGCTCAAGAACCGCAGGCTC   240
241  ACGTGGCAGTACTGTGATGTGCCCTCCTGCTCCACCTGCGGCCTCAGACAGTACAGCCAC   300
301  CCTCACTTTCGCATCAAAGGACGGCTCTTCCCCGACATCGCCCTCCCACCCCTCCCACCCT   360
361  CCCATCTTTCCCAACCACACCACCTCCCCCCCGAGAGCCGCTTCCTGTGCCCCGCCCATACTC   420
421  ATCAGCTCCTCCTCCATTCTCTCTCCCGCCCACTGCTTCCAGGAGACCGTTTCCGCCCCAC   480
481  CACCTCACCCTCATCTTCGGCACAACATACCGGCTGGTCCCTCGCCAGCACCACCACAAA   540
541  TTTGAAGTCCAAAAATACATTGTCCATAACCAATTCCATCATCACACTTACGACAATCAC   600
601  ATTCCCCTCCTCCAGCCTCAAATCGCATTCGTCCCCCTCTCCCCAGCACACCAGCGTCCTC   660
661  CCCACTCTCTGCCTTCCCCCCGCCCGACCTGCACGTGCCGGACTCGACGGAGTGTCAGCTC   720
721  TCCGGCTACGGCAAGCATCACGCCTTGTCTCCTTTCTATTCCCACCGGCTCAACGAGCCT   780
781  CATGTCAGACTGTACCCATCCAGCCGCTCCACATCACAACATTTACTTAACAGAACACTC   840
841  ACCGACAACATGCTGTGTGCTGGAGACACTCCCACCGCCGCGCCCCAGGCAAACTTGCAC   900
901  GACCCCTCCCAGCCCCATTCGCCAGCCCCCCTCGTGTCTCTGAACGATGGCCCCATGACT   960
961  TTGGTGCGCATCATCAGCCTCGGCCCTCGGGCTCTGCACAGCAACCATCTCCCCGGTGTCTAC  1020
1021 ACAAAGGTTACCAAGCTACCTAGACTGCATTCGTGACAACATGGCGACGG  1068
```

or a DNA sequence which differs therefrom within the scope of the degeneracy of the genetic code which codes for a t-PA derivative which is not glycosylated and has the following amino acid sequence

```
(M)

  1  SYQCNSDCYF  CNCSAYRCTH  SLTESGASCL  PWNSHILICK  VYTAQNPSAQ

 51  ALGLCKHNYC  RNPDGDAKPW  CHVLXNRRLT  WEYCDVPSCS  TCGLRQYSQP

101  QFRIKCCLFA  DIASHPWQAA  IFAXHRRSPC  ERFLCCGILI  SSCWILSAAH

151  CFQERFPPHH  LTVILCRTYR  VVPGEEEQKF  EVEKYIVHKE  FDDDTYDNDI

201  ALLQLKSDSS  RCAQESSVVR  TVCLPPADLQ  LPDWTECELS  CYCKHEALSP

251  FYSERLKEAH  VRLYPSSRCT  SQHLLNRTVT  DNMLCACDTR  SGCPQANLHD

301  ACQCDSGGPL  VCLNDCRMTL  VGIISWGLCC  CQKDVPGVYT  KVTNYLDWIR

351  DNMRP
```

or for the production of plasmid pA27.3 according to Figure 1 or pA27fd prepared according to example 5,

**wherein**

a DNA sequence which codes for the whole t-PA protein or a derivative thereof containing further regions of the t-PA protein in addition to the kringle II and the protease domains is incorporated into a plasmid and those regions which code for amino acids which are not present in the t-PA derivative are deleted by site-directed mutagenesis.

**12.** Process as claimed in claim 11,

**wherein**

the corresponding cDNA is used as the DNA sequence for the t-PA protein or a derivative thereof.

## Revendications

**Revendications pour les Etats contractants suivants : AT, BE, CH, LI, DE, DK, FR, GB, IT, LU, NL, SE**

**1.** Dérivé de l'activateur tissulaire du plasminogène (t-PA), caractérisé en ce qu'il n'est pas glycosylé et consiste en la séquence d'acides aminés suivante:

```
(M)

  1  SYQCNSDCYF  CNCSAYRCTH  SLTESGASCL  PWNSHILICK  VYTAQNPSAQ

 51  ALGLCKHNYC  RNPDGDAKPW  CHVLXNRRLT  WEYCDVPSCS  TCGLRQYSQP

101  QFRIKCCLFA  DIASHPWQAA  IFAXHRRSPC  ERFLCCGILI  SSCWILSAAH

151  CFQERFPPHH  LTVILCRTYR  VVPGEEEQKF  EVEKYIVHKE  FDDDTYDNDI

201  ALLQLKSDSS  RCAQESSVVR  TVCLPPADLQ  LPDWTECELS  CYCKHEALSP

251  FYSERLKEAH  VRLYPSSRCT  SQHLLNRTVT  DNMLCACDTR  SGCPQANLHD

301  ACQCDSGGPL  VCLNDCRMTL  VGIISWGLCC  CQKDVPGVYT  KVTNYLDWIR

351  DNMRP
```

qui peut être prolongée encore par M à l'extrémité amino.

**2.** Séquence d'ADN caractérisée en ce qu'elle code un dérivé du t-PA selon la revendication 1 et contient la séquence suivante:

```
   1  ATGTCTTACCAAGCAAACACTGACTGCTACTTTGGCAATGGGTCAGCCTACCGTGGCACC .   60
  61  CACAGGGTGACGGAGTCGGGTGCCTGCTGGCTCGGGTGGAATTGCATGATGGTCGATAGGC  120
 121  AAGGTTTACACAGGACAGAACCCGAGTGCCCAGGCAGTGGGGCCTGGGCAAACATAATTAC  180
 181  TGGGGCAATGGTGATGGGGATGGGAAGGGGTGGTGGGACGTGGTGAAGAACGGGAGGGTG   240
 241  ACGTGGGAGTAGTGTGATGTGGGGTGGTGGTGGACGTGGGGGGTGAGACAGTACAGGGAG  300
 301  CGTGAGTTTCGGCATGAAAGGAGGGGTGTTGGGGGACATGGGGTGGGACCGGTGGGAGGGT  360
 361  GGGATGTTTGGGAAGGACAGGAGGTGGGGGGGAGAGGGGTTGGTGTGGGGGGGGGCATAGTG  420
 421  ATGAGGTGGTGGTGGATTGTGTGTGGGGGGGGCAGTGGTTGGAGGAGAGGTTTGGGGGGCAG  480
 481  CAGGTGAGGGTGATGTTGGGGGAGAAGATAGGGGGTGGTGGGTGGGGAGGAGGAGGAGAAA  540
 541  TTTGAAGTGGAAAAATAGATTGTGGATAAGGAATTGGATGATGAGAGTTAGGAGAAATGAG  600
 601  ATTGGGGTGGTGGAGGTGAAATGGGATTGGTGGGGGTGTGGGGAGGAGAGGAGGGTGGTG  660
```

```
 661  GGGAGTGTGTGGGTTGGGGGGGGGGGAGGTGGAGGTGGGGGGAGTGGAGGGAGTGTGAGGTG  720
 721  TGGGGGTAGGGGAAGGATGAGGGGTTGTGTGGTTTGTATTGGGAGGGGGTGAAGGAGGGT  780
 781  GATGTGAGAGTGTAGGGATGGAGGGGGTGGACATGAGAAGATTTAGTTAAGAGAAGAGTG  840
 841  AGGGAGAAGATGGTGTGTGGTGGAGAGAGTGGGAGGGGGGGGGGGGGGGGGGGCAAAGTTGGAG .  900
 901  GAGGGGTGGGAGGGGGATTGGGGAGGGGGGGTGGTGTGTGTGAAGGATGGGGGGATGAGT   960
 961  TTGGTGGGGATGATGAGGTGGGGGGGTGGGGTGTGGAGAGAAGGATGTGGGGGGTGTGTAG  1020
1021  ACAAAGGTTAGGAAGTAGGTAGAGTGGATTGGTGAGAAGATGGGAGGG  1068
```

**3.** Plasmide d'expression caractérisé en ce qu'il contient la séquence d'ADN selon la revendication 2, ou une séquence d'ADN différente de celle-ci dans le cadre de la dégénérescence du code génétique, qui code une protéine selon la revendication 1.

**4.** Plasmide pA27.3 contenant une séquence d'ADN selon la revendication 2 et représenté schématiquement sur la figure 1.

**5.** Plasmide pA27fd contenant une séquence d'ADN selon la revendication 2 ligaturée dans un vecteur qui est présent en un grand nombre de copies dans des cellules hôtes et qui est préparé de la manière décrite dans l'exemple 5.

**6.** Procédé de production d'un plasmide selon l'une des revendications 3 à 5, caractérisé en ce que l'on introduit dans un plasmide une séquence d'ADN qui code la protéine t-PA totale ou un dérivé de celle-ci qui, outre les domaines kringel II et de protéase, présente encore d'autres domaines de la protéine t-PA et on élimine par mutagenèse dirigée les domaines qui codent des acides aminés qui ne sont pas présents dans le dérivé du t-PA selon la revendication 1.

**7.** Procédé selon la revendication 6, caractérisé en ce que l'on utilise l'ADNc correspondant comme séquence d'ADN pour la protéine t-PA ou un dérivé de celle-ci.

**8.** Procédé selon la revendication 6 ou 7, caractérisé en ce que l'on utilise le plasmide pA27.3 selon la revendication 4 ou le plasmide pA27fd selon la revendication 5.

28

**9.** Procédé de production d'un dérivé du t-PA selon la revendication 1, caractérisé en ce que l'on transforme des cellules hôtes appropriées avec un plasmide selon l'une des revendications 3 à 5 et on recueille le produit d'expression à partir du milieu de culture ou après lyse des cellules hôtes.

**10.** Procédé selon la revendication 9, caractérisé en ce que l'on utilise comme cellules hôtes des cellules procaryotes, en particulier E. coli.

**11.** Procédé selon la revendication 10, caractérisé en ce que, pour augmenter le rendement en protéine active, on sépare les "inclusion bodies" formés, on les solubilise par traitement avec le chlorhydrate de guanidine, puis on les transforme en dérivé avec du glutathion oxydé et enfin on renature le dérivé du t-PA par addition de L-arginine et de GSH.

**12.** Procédé selon la revendication 11, caractérisé en ce que, après la renaturation, on concentre K2P dans le mélange de renaturation puis on réalise une purification chromatographique par chromatographie d'affinité.

**13.** Procédé selon la revendication 12, caractérisé en ce que l'on opère en présence de 10 à 1000 mmol/l de L-arginine.

**14.** Procédé selon la revendication 13, caractérisé en ce que l'on réalise la purification chromatographique avec le concentré du mélange de renaturation qui contient 10 à 1000 mmol/l, de préférence 600 à 800 mmol/l de L-arginine, sur une colonne de ETI-adsorbant.

**15.** Procédé selon la revendication 14, caractérisé en ce que l'on ajuste à un pH de 7,5 à 8,6 le concentré utilisé pour la purification chromatographique.

**16.** Procédé selon la revendication 14 ou 15, caractérisé en ce que l'on réalise l'élution à un pH de 3 à 5,5.

**17.** Médicament pour le traitement fibrinolytique contenant un dérivé du t-PA selon la revendication 1.

**Revendications pour les Etats contractants suivants : ES, GR**

**1.** Procédé de préparation d'un dérivé de l'activateur tissulaire du plasminogène (t-PA) qui n'est pas glycosylé et qui consiste en la séquence d'acides aminés suivante:

```
                (M)

          1   SYQGNSDCYF GNGSAYRGTH SLTESGASCL PWNSMILIGK VYTAQNPSAQ

         51   ALGLGKHNYC RNPDGDAKPW CHVLKNRRLT WEYCDVPSCS TCGLRQYSQP

        101   QFRIKGGLFA DIASHPWQAA IFAKHRRSPG ERFLCGGILI SSCWILSAAH

        151   CFQERFPPHH LTVILGRTYR VVPGEEEQKF EVEKYIVHKE FDDDTYDNDI

        201   ALLQLKSDSS RCAQESSVVR TVCLPPADLQ LPDWTECELS GYGKHEALSP

        251   FYSERLKEAH VRLYPSSRCT SQHLLNRTVT DNMLCAGDTR SGGPQANLHD

        301   ACQGDSGGPL VCLNDGRMTL VGIISWGLGC GQKDVPGVYT KVTNYLDWIR

        351   DNMRP
```

qui peut être prolongée encore par M à l'extrémité amino, caractérisé en ce que l'on introduit dans des cellules hôtes appropriées un plasmide d'expression qui contient la séquence d'ADN:

```
   1  ATGTCTTACCAACGAAACACTGACTGCTACTTTCGGAATCGGTCAGCCTACCGTGCCACG .   60
  61  CACAGCCTCACCGAGTCGGGTGCCTCCTGCCTCCCGTGGAATTCCATCATCCTCATAGCC   120
 121  AAGGTTTACACAGCACAGAACCCCAGTGCCCAGGCACTGGGCCTCGGCAAACATAATTAC   180
 181  TGCCCGAATCCTCATCGGGATCCCAAGCCCTCGTGCCACGTGCTCAAGAACCGCAGGCTG   240
 241  ACGTGCCAGTACTGTCATGTGCCCCTCCTGCTCCACCTGCCGCCCTGAGACAGTACAGCCAG   300
 301  CCTCAGTTTCCCATCAAAGGAGCCCTCTTCGCCCGACATCGCCCTCCCACCCCTGGCAGGCT   360
 361  CCCATCTTTGCCAACCACAGGACGTCGCCCCCCAGAGCCGGTTCCTGTGCCGGCGGCATACTC   420
 421  ATCAGCTCCTGCTGCATTCTCTCTGCCGCCCCACTGCTTCCAGCACACGTTTCCCCCCCAC   480
 481  CACCTGACCGTGATCTTCGGGCAGAACATACCGGGTGGTCCCTGCCCAGGACCACCACAAA   540
 541  TTTGAAGTCGAAAAATACATTGTCCATAAGGAATTCGATGATGACACTTACCACAATGAC   600
 601  ATTCCGCTGCTGCAGCTGAAATCGCATTCGTCCCGCTGTGCCCAGGACAGCACCGTCGTG   660
```

```
 661  CGCACTGTGTGCCCTTCCCCCGGCCGACCTGCAGCTGCCCGGACTGGACGGAGTGTGACCTG   720
 721  TGCGGCTACGGCAAGCATGACGCCCTTGTGTGTGCTCCTTTTCTATTCGGAGCCGGCTCAAGGAGGCT   780
 781  CATGTGCAGACTGTACCCATGCAGGCCGGCTGCACATCACAACATTTACTTAACAGAACAGTG   840
 841  ACGGACAACATGCTGTGTGCTGGCAGACACTCGGCAGCCGGCCGGGCCCCAGGCAAACTTGGCAC   900
 901  GACGGCCTGCCAGGGGCCGATTCGGGCAGCCCCCCTGGTGTCTGTGTCTGAACGATCGGGCCCCATGACT   960
 961  TTGGTGGGCATCATCAGGCTGGGGGGCCTCGGGCTGTGGACAGAAGGATGTCCCCGGGTGTGTAC  1020
1021  ACAAAGGTTACCAACTACCTAGACTGGATTCGTGACAACATGGGACCG  1068
```

ou une séquence d'ADN différente de celle-ci dans le cadre de la dégénérescence du code génétique, qui code un dérivé du t-PA correspondant et on recueille le produit d'expression à partir du milieu de culture ou après lyse des cellules hôtes.

2. Procédé selon la revendication 1, caractérisé en ce que l'on utilise le plasmide pA27.3 représenté schématiquement sur la figure 1.

3. Procédé selon la revendication 1, caractérisé en ce que l'on utilise le plasmide pA27fd préparé de la manière décrite dans l'exemple 5.

4. Procédé selon la revendication 1, 2 ou 3, caractérisé en ce que l'on utilise comme cellules hôtes des cellules procaryotes, en particulier E. coli.

5. Procédé selon la revendication 4, caractérisé en ce que, pour augmenter le rendement en protéine active, on sépare les "inclusion bodies" formés, on les solubilise par traitement avec le chlorhydrate de guanidine, puis on les transforme en dérivé avec du glutathion oxydé et enfin on renature le dérivé du t-PA par addition de L-arginine et de GSH.

6. Procédé selon la revendication 5, caractérisé en ce que, après la renaturation, on concentre le dérivé du t-PA dans le mélange de renaturation puis on réalise une purification chromatographique par chromatographie d'affinité.

**7.** Procédé selon la revendication 6, caractérisé en ce que l'on opère en présence de 10 à 1000 mmol/l de L-arginine.

**8.** Procédé selon la revendication 7, caractérisé en ce que l'on réalise la purification chromatographique avec le concentré du mélange de renaturation qui contient 10 à 1000 mmol/l, de préférence 600 à 800 mmol/l de L-arginine, sur une colonne de ETI-adsorbant.

**9.** Procédé selon la revendication 8, caractérisé en ce que l'on ajuste à un pH de 7,5 à 8,6 le concentré utilisé pour la purification chromatographique.

**10.** Procédé selon la revendication 8 ou 9, caractérisé en ce que l'on réalise l'élution à un pH de 3 à 5,5.

**11.** Procédé de préparation d'un plasmide d'expression qui contient la séquence d'ADN:

```
   1  ATGTCTTACCAAGGAAACACTGACTGCTACTTTGGGAATGGGTCAGCCTACCGTGCCACC  60
  61  CACAGCCTCACCGACTCGGGTGCCTCCTGCCTCCCGTGGAATTCCATGATCCTGATAGGC  120
 121  AAGGTTTACACAGCCACAGAACCCCAGTGCCCAGGCACTGGCCCTGGGCAAACATAATTAC  180
 181  TGCCCGAATCCTGATCGGGCATGCCAAGCCCTGGTGCCACGTGCTGAAGAACCGCAGCCTG  240
 241  ACGTGGCAGTACTGTGATGTGCCCTCCTGCTCCACCTGCGGCCTGAGACAGTACAGCCAG  300
 301  CCTGACTTTGGCATCAAAGGAGCGGCTCTTCGCCCGACATGCCCTCCCACCCCTGGCAGGCT  360
 361  GGCATCTTTGGCAAGGCACAGGAGGTGGCCCCGGAGAGCCGGTTCCTGTGCGGCGGCATACTC  420
 421  ATCAGCTCCTGGTGGATTCTGTCTGCCGGGCCCACTGGTTGGACGAGACGGTTTGCGGCCCCAC  480
 481  CACGTGACGGTGATCTTGGGCAGAACATACGGGGTGGTCCCTGGCGAGGAGGAGGAGAAA  540
 541  TTTGAAGTGGCAAAAAATACATTGTGCCATAAGGAATTCGATGATGACACTTACCACAATGAC  600
 601  ATTGGGCTGGCTGGCAGGCTCAAATGGGCATTGGTGCCCGGCTGTGGCCCAGGACAGCAGCCTGGTG  660
 661  GGGCACTGTGTGGGCCTTGCCGGGCCGGGGACCTGGCAGGCTGGCCCGGACTGGACGGGAGTGCTGGCAGGCTGG  720
 721  TGGGGCTACGGCAAGGCATGAGGGCCTTGTCTGCCTTTTCTATTGGGCAGCCGGGCTCAAGGGACGGCT  780
 781  CATGTGCAGACTGTACCCATGGCAGGCCCCGCTGGCACATGCACAACATTTACTTAACAGAAGCAGTGC  840
 841  ACCGACAACATGGCTGTGTGCTGGCAGACACTGGCAGGCGGGCGGGCGGGCCCCAGGCAAACTTGGCAC  900
 901  GACGGGCTGCCCAGGGGCCGATTGGGGGAGGGCCCCCTGGTCTGTCTGAACGATGGGCCCCATGACT  960
 961  TTGGTGGGGCATCATCAGCTGGGGGCCCTGGGCCTGTGGGACAGAAGGATGTGCCCCGGGTGTCTAC  1020
1021  ACAAAGGTTACCAACTACCTAGACTGGATTCGTGACAACATGGCGACCG  1068
```

ou une séquence d'ADN différente de celle-ci dans le cadre de la dégénérescence du code génétique, qui code un dérivé du t-PA qui n'est pas glycosylé et qui présente la séquence d'acides aminés suivante:

(M)

```
  1  SYQGNSDCYF GNGSAYRGTH SLTESGASCL PWNSMILIGK VYTAQNPSAQ
 51  ALGLGKHNYC RNPDGDAKPW CHVLKNRRLT WEYCDVPSCS TCGLRQYSQP
101  QFRIKGGLFA DIASHPWQAA IFAKHRRSPG ERFLCGGILI SSCWILSAAH
151  CFQERFPPHH LTVILGRTYR VVPGEEEQKF EVEKYIVHKE FDDDTYDNDI
201  ALLQLKSDSS RCAQESSVVR TVCLPPADLQ LPDWTECELS GYGKHEALSP
251  FYSERLKEAH VRLYPSSRCT SQHLLNRTVT DNMLCAGDTR SGGPQANLHD
301  ACQGDSGGPL VCLNDGRMTL VGIISWGLGC GQKDVPGVYT KVTNYLDWIR
351  DNMRP
```

ou de préparation du plasmide pA27.3 selon la figure 1 ou pA27fd préparé selon l'exemple 5, caractérisé en ce que l'on introduit dans un plasmide une séquence d'ADN qui code la protéine t-PA totale ou un dérivé de celle-ci qui, outre les domaines kringel II et de protéase, présente encore d'autres domaines de la protéine t-PA et on élimine par mutagenèse dirigée les domaines qui codent des acides aminés qui ne sont pas présents dans le dérivé du t-PA.

12. Procédé selon la revendication 11, caractérisé en ce que l'on utilise l'ADNc correspondant comme séquence d'ADN pour la protéine t-PA ou un dérivé de celle-ci.

# FIG. 1

pREM 7685
(FK2P)

pACYC 177

TM

Ap$^R$

EcoRI

EcoRI

EcoRI

P$_{tac}$

XhoI

Km$^R$

Deletion der
Finger-Domäne

Mutagenese-Primer
5'TGTCTTACCAAGGAAACAGTGA3'

pA 27.3
(K2P)

TM

Ap$^R$

EcoRI

EcoRI

EcoRI

P$_{tac}$

XhoI

Km$^R$

Finger-Domäne (F)

Kringel II-Domäne (K2)

Protease-Domäne (P)

TM = Terminator
P$_{tac}$ = tac-Promoter
Ap$^R$ = Ampicillin-Resistenz
Km$^R$ = Kanamycin-Resistenz

FIG.2

FIG.3

EP 0 382 174 B1

FIG. 4

# FIG.5

FIG.6

EP 0 382 174 B1

FIG.7